# EUROPEAN PATENT APPLICATION

(11) **EP 3 415 630 A1**
(43) Date of publication of application: **19.12.2018**
(21) Application number: 17382366.7
(22) Date of filing: 14.06.2017
(51) Int. Cl.: C12P 13/00

(54) **METHOD FOR ONE-POT PRODUCTION OF A PRIMARY AMINE FROM AN ALCOHOL**

(71) Applicant: EntreChem, S.L., 33006 Oviedo (ES)
(72) Inventor: Liardo, Elisa, 33006 Oviedo (ES); Ríos Lombardía, Nicolás, 33006 Oviedo (ES); González Sabín, Javier, 33006 Oviedo (ES); Morís Varas, Francisco, 33006 Oviedo (ES)
(74) Representative: Manuel Illescas y Asociados, S.L.U.

(57) **Abstract**

A method for producing a compound of formula (III) 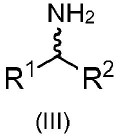 or a salt thereof, wherein said process comprises the following steps:
(a) forming a mixture comprising a compound of formula (II) 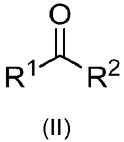 by adding an organocatalyst to a compound of formula (I) 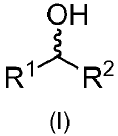 in an aqueous medium comprising an oxidizing agent and an organic co-solvent; and
(b) adding an aqueous buffer, a transamination catalyst, an amine donor compound and a cofactor to the mixture obtained in step (a), wherein said transamination catalyst is added at the same time as or after adding said aqueous buffer.

## Description

### Field of the invention

The present invention relates to a method for producing an amine, or a salt thereof, from an alcohol. The present invention also relates to an amine, or a salt thereof, which is obtained from said method.

### Background of the invention

Enantiomerically pure chiral amines are of increasing value in organic synthesis, for instance as resolving agents, chiral auxiliaries/chiral bases, and catalysts for asymmetric synthesis. Moreover, optically active amines are also valuable building blocks for the preparation of a broad range of pharmaceuticals. Consequently, there is a need for efficient methods to obtain both enantiomers in optically pure form from readily-available starting materials.

No enzyme is known to exist which is capable of catalyzing the direct transformation of alcohols to amines, although several multi-enzymatic approaches have been developed for effecting this. In particular, US 2014/0120587 A1 discloses a selective biocatalytic two-step oxidation-reductive amination sequence consisting of an alcohol dehydrogenase (ADH)-catalyzed oxidation of a racemic alcohol and further amination mediated by an omega-transaminase. However, this sequence usually requires the use of two enzymes with opposite stereopreferences due to the excellent selectivities commonly shown by ADH enzymes when a racemic alcohol is used. In addition, alcohol oxidases have been also employed in conjunction with omega-transaminases for transforming cinnamic and benzylic alcohols (via a two-enzyme cascade) and also non-activated aliphatic alcohols (via a five enzyme-cascade) into the corresponding amines [a) RSC Adv. 2012, 2, 6262-6265; b) ChemCatChem 2015, 7, 3121-3124]. Moreover, laccases have also been recently employed as non-stereoselective oxidants in conjunction with omega-transaminases, but require a high concentration (>30 mol%) of a chemical mediator such as TEMPO and are only applicable to benzylic alcohols [Green Chem. 2017, 19, 474-480]. However, all of the aforementioned methodologies are limited to exclusively enzyme-mediated processes.

In fact, the use of enzymes in synthesis is limited since only a few classes of enzymes are compatible with common organic solvents.

Therefore, a problem underlying the present invention is to provide a method that may be used to produce amines starting from compounds comprising a hydroxyl group, preferably attached to an aromatic or aliphatic hydrocarbon.

Another problem underlying the present invention is to provide a chemoenzymatic method for the production of enantiomerically enriched or enantiomerically pure organic amines, wherein said route has a broad substrate specificity and/or improved yield and/or is more economical compared to reactions described in the prior art. Preferably, said methods do not involve hazardous reagents or harsh reaction conditions.

A further problem underlying the present invention is to provide a method for producing enantiomerically and/or diastereomerically enriched or enantiomerically and/or diastereomerically pure organic amino alcohols or diamines, starting from compounds comprising a hydroxyl group, preferably attached to an aromatic or aliphatic hydrocarbon.

Moreover, another problem underlying the present invention is to provide a system that may be used to produce amines starting from sterically hindered secondary alcohols, which are typically difficult to oxidize.

These problems are solved according to the present invention.

### Brief description of the invention

The present invention relates to a method for producing a compound of formula (III) 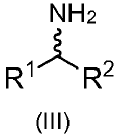 or a salt thereof, wherein said method comprises the following steps:
(a) forming a mixture comprising a compound of formula (II) 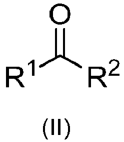 by adding an organocatalyst to a compound of formula (I) 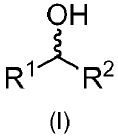 in an aqueous medium comprising an oxidizing agent and an organic co-solvent; and
(b) adding an aqueous buffer, a transamination catalyst, an amine donor compound and a cofactor to the mixture obtained in step (a), wherein said transamination catalyst is added at the same time as or after adding said aqueous buffer,
wherein:
(i) R¹ is an R³ substituent and R² is an R³ substituent, wherein each R³ substituent is independently selected from:
   - an R^{3a} substituent;
   - an R^{3b} substituent; or
   - an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with
      - an R^{3a} substituent,
      - an R^{3b} substituent, or
      - an R^{3a} substituent that is optionally substituted with an R^{3b} substituent,
      or
(ii) R¹ and R², when taken with the carbon atom to which they are both attached, complete a non-aromatic 5-membered to 8-membered ring of a carbocycle or a heterocycle comprising at least one heteroatom selected from O, N or S, wherein said carbocycle or heterocycle comprises one ring or two to four fused 5-membered to 8-membered rings, and wherein said carbocycle or heterocycle is unsubstituted or substituted at one or more atoms of N, C or S with 1 to 4 R³ substituents, wherein each R³ substituent is independently selected from:
   - an R^{3a} substituent;
   - an R^{3b} substituent;
   - an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with an R^{3b} substituent;
   - =O;
   - =NR⁴; or
   - =S,
and wherein:
- each R^{3a} substituent is independently selected from an alkyl, an alkenyl, an alkynyl, a carbocyclyl or a heterocyclyl moiety comprising at least one heteroatom selected from O, N or S, wherein said carbocyclyl or a heterocyclyl moiety comprises one to four fused 5-membered to 8-membered rings;
- each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN, -Si(R⁵)₃, -SR⁵, -(S=O)-R⁵, -(S=NR⁴)-R⁵, -S(=O)₂-R⁵, -S(=O)₂-NR⁴R⁶ or -S(=O)(=NR⁴)-R⁵;
- R⁴ is H or R⁶;
- R⁵ is H or an R^{3a} substituent that is optionally substituted with another R^{3a} substituent;
- R⁶ is an R^{3a} substituent or -(C=O)-OR⁵; and
- Z is halogen.

The present invention also relates to a method for producing a compound of formula (IIIa) 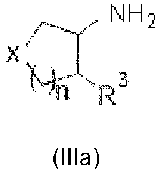 or a salt thereof, wherein said method comprises the following steps:
(a) forming a mixture comprising a compound of formula (IIa), 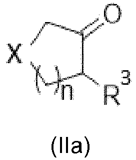 by adding an organocatalyst to a compound of formula (Ia), 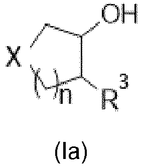 in an aqueous medium comprising an oxidizing agent and an organic co-solvent; and
(b) adding an aqueous buffer, an omega-transaminase enzyme, an amine donor compound and a cofactor to the mixture obtained in step (a), wherein said omega-transaminase enzyme is added at the same time as or after adding said aqueous buffer,
wherein
the compound of formula (IIIa) is formed by dynamic kinetic resolution and is enriched in one enantiomer and/or enriched in at least one diastereomer thereof,
adding the buffer to the mixture obtained in step (a) dilutes said mixture at least two-fold, and step (b) is carried out at a pH of between 9 and 12,
n represents a whole number selected from 1, 2 or 3,
X is selected from CR⁵R⁵, CR⁵R⁶, O, SO₂, S, NH or NR⁴, , and
R³ is selected from:
- an R^{3a} substituent;
- an R^{3b} substituent;
- an R^{3a} substituent which is substituted with 1 or 2 substituents each independently selected from =O, -Z, an R^{3a} substituent or an R^{3b} substituent,
and wherein:
each R^{3a} substituent is independently selected from a C₁-C₈ alkyl, a C₅-C₈ monocyclic cycloalkyl, a monocyclic or bicyclic C₅-C₁₀ aryl comprising one or two aromatic rings or a monocyclic or fused-bicyclic 5 to 9-membered heterocyclyl moiety comprising at least one heteroatom selected from O, N or S; and
each R^{3b} substituent is independently selected from -OR⁵, -(C=O)-R⁵, -(C=O)-OR⁵, -(C=O)-NR⁴R⁶, -O(C=O)-OR⁵, -NR⁴R⁶, -CN, -Si(R^{3a})₃,-(S=O)-R^{3a};
   wherein
R⁴ is H or R⁶;
each R⁵ substituent is independently selected from H, an R^{3a} substituent or an R^{3a} substituent that is substituted with another R^{3a} substituent;
R⁶ is an R^{3a} substituent or -(C=O)-OR⁵; and
Z is F or Cl.

### Figures

The following figures illustrate the present invention and should not be construed as limiting said invention in any way. The substituents and integers used in the said figures are as defined in the embodiments of the present invention, unless otherwise indicated.
Figure 1: a general scheme of the one-pot, two-step process for the stereoselective transformation of racemic alcohols into amines through sequential (*i.e.* stepwise) action of an organocatalyst and a transaminase as per the method according to the present invention.
Figure 2: scheme of the process for the conversion of a racemic mixture of compound (1) [*i.e.* of Formula (II)] to the amine products of compounds (2a), (2b), (2c) and/or (2d) [*i.e*. of Formula (III)].
Figure 3: scheme of the process for the transformation of (3) [*i.e*. of Formula (II)] into compound (4a), (4b), (4c) and/or (4d) [*i.e*. of Formula (III)].
Figure 4: scheme of the process for the deprotection of amines.
Figure 5: a representation of aliphatic and aromatic alcohols [*i.e.* of Formula (I)] which were aminated according to the one-pot, two-step sequential (*i.e.* stepwise) process in scheme of Figure 1.
Figure 6: a representation of β-substituted cycloalkanols bearing two chiral centers [*i.e.* of Formula (I)], which were aminated according to the one-pot, two-step sequential (*i.e.* stepwise) process in scheme of Figure 1.
Figure 7: A. HPLC analysis of *N*-acetyl derivatives prepared from standards of *cis*-(±)- and *trans-*(±)-2-(benzyloxy)cyclohexanamine, and B. an example of in-process HPLC monitoring for the bioamination of (±)-2-(benzyloxy)cyclohexanone with an omega-transaminase according to the present invention.
Figure 8: NMR data of (±)-2-(benzyloxy)cyclohexanol, a starting material employable in the method of present invention.
Figure 9: NMR data of (±)-2-(benzyloxy)cyclohexanone, the product resulting from the oxidation of (±)-2-(benzyloxy)cyclohexanol with an 2-azaadamantane-N-oxyl (AZADO)/NaOCI organocatalyst/oxidant system according to the present invention.
Figure 10: NMR data of *cis*-2-(benzyloxy)cyclohexanamine, the product resulting from the stereoselective transformation of (±)-2-(benzyloxy)cyclohexanol through the stepwise action of an organocatalyst and a transaminase in the method according to the present invention.
Figure 11: NMR data of *trans*-2-(benzyloxy)cyclohexanamine, the product resulting from the stereoselective transformation of (±)-2-(benzyloxy)cyclohexanol through the stepwise action of an organocatalyst and a transaminase in the method according to the present invention.
Figure 12: in-process HPLC monitoring for the stereoselective transformation of (±)-1-(3-(trifluoromethyl)phenyl)propan-2-ol into optically active 1-(3-(trifluoromethyl)phenyl)propan-2-amine through the stepwise action of an organocatalyst and a transaminase in the method according to the present invention (A. to C.) followed by subsequent acetylation (D.) in order to allow enantiomeric excess to be measured.
Figure 13: in-process HPLC monitoring for the stereoselective transformation of (±)-2-(benzyloxy)cyclohexanol into optically active 2-(benzyloxy)cyclohexanamine through the stepwise action of an organocatalyst and a transaminase in the method according to the present invention (A. to C.).

### Detailed description of the invention

The present invention relates to a method for producing a compound of formula (III) 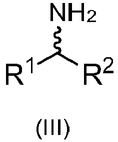 or a salt thereof from a compound of formula (I)

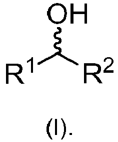

Said method is thus a process of manufacture of a compound of formula (III) or a salt thereof from a compound of formula (I). The compound of formula (I) is a secondary alcohol. The compound of formula (III) is a primary amine. The salt of said primary amine is preferably selected from a hydrochloride, hydrobromide, hydrogen sulfate, hydrogen phosphate, hydrogen citrate or hydrogen oxalate salt. The present invention also relates to a process of manufacture of a formate, acetate, propionate or benzoate ester by respective formylation, acetylation, propionylation or benzoylation of a compound of formula (III), when the compound of formula (III) comprises a hydroxyl moiety.

The method for producing a compound of formula (III) of the present invention comprises the following steps:
(a) forming a mixture comprising a compound of formula (II) 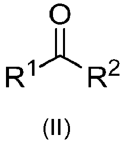 by adding an organocatalyst to a compound of formula (I) in an aqueous medium comprising an oxidizing agent and an organic co-solvent; and
(b) adding an aqueous buffer, a transamination catalyst, an amine donor compound and a cofactor to the mixture obtained in step (a), wherein said transamination catalyst is added at the same time as or after adding said aqueous buffer.

The compound of formula (II) is a ketone.

Thus, step (a) comprises oxidizing the compound of formula (I) to form a mixture comprising a compound of formula (II). Said oxidation is performed by an organocatalyst. Said organocatalyst is a compound which acts as an "electron shuttle" or mediator of the oxidation in that it is first oxidized by the oxidizing agent, and the resulting oxidized compound in turn oxidizes the compound of formula (I), before being reduced back to the organocatalyst.

Preferably, the organocatalyst is a nitroxyl compound, a hydroxyamino compound or an azine compound. In one more preferred embodiment of the present invention, the organocatalyst is selected from 2,2,6,6-tetramethylpiperidin-1-oxyl, 4-substituted derivatives of 2,2,6,6-tetramethylpiperidin-1-oxyl, 9-azabicyclo[3.3.1]nonane-*N-*oxyl (ABNO), 9-azabicyclo[3,3,1]nonan-3-one-9-oxyl (keto-ABNO), 2,2'-azino-bis(3-ethylbenzothiazoline-6-sulphonic acid) (ABTS), VA (violuric acid), 2-azaadamantane-*N*-oxyl (AZADO), 1-methyl-2-azaadamantane-*N*-oxyl (1-methyl AZADO), 1,3-dimethyl-2-azaadamantane-*N*-oxyl (1,3-dimethyl AZADO) or *N*-hydroxyphthalimide [the phthalimide *N-*oxyl (PINO) radical organoicatalyst being formed therefrom by activation *in situ*]*.* Preferably, said 4-substituted derivatives of 2,2,6,6-tetramethylpiperidin-1-oxyl include 4-hydroxy-2,2,6,6-tetramethylpiperidin-1-oxyl (4-hydroxy TEMPO), 4-amino-2,2,6,6-tetramethylpiperidin-1-oxyl (4-amino TEMPO), 4-carboxy-2,2,6,6-tetramethylpiperidin-1-oxyl (4-carboxy TEMPO), 4-acetamido-2,2,6,6-tetramethylpiperidine-1-oxyl (4-acetamido TEMPO), 4-oxo-2,2,6,6-tetramethylpiperidin-1-oxyl (4-oxo TEMPO), 4-methoxy-2,2,6,6-tetramethylpiperidin-1-oxyl (4-methoxy TEMPO), 4-ethoxy-2,2,6,6-tetramethylpiperidin-1-oxyl (4-ethoxy TEMPO), 4-hydroxy-2,2,6,6-tetramethylpiperidin-1-oxyl benzoate, 4-hydroxy-2,2,6,6-tetramethylpiperidin-1-oxyl ethanoate, 4-hydroxy-2,2,6,6-tetramethylpiperidin-1-oxyl methanoate, 4-carboxy-2,2,6,6-tetramethylpiperidin-1-oxyl, (2-(2,2,6,6-tetramethylpiperidin-1-oxyl-4-ylamino)-2-oxoethyl)triphenylphosphonium chloride (Mito-TEMPO), 4-isothiocyanato-2,2,6,6-tetramethylpiperidine 1-oxyl (4-isothiocyanato TEMPO), 4-(2-bromoacetamido)-2,2,6,6-tetramethyl-1-piperidinyloxy, 4-(2-iodoacetamido)-2,2,6,6-tetramethyl-1-piperidinyloxy, 4-(2-chloroacetamido)-2,2,6,6-tetramethyl-1-piperidinyloxy, 2,2,6,6-tetramethyl-4-[1-oxo-6-(triethylammonio)hexylamino]-1-piperidinyloxy bromide (WS TEMPO), 4-phosphonooxy-2,2,6,6-tetramethyl-1-piperidinyloxy hydrate (4-phosphonooxy-TEMPO hydrate), 2,2,6,6-tetramethyl-4-(methylsulfonyloxy)-1-piperidinooxy, 4-maleimido-2,2,6,6-tetramethyl-1-piperidinyloxy (4-maleimido TEMPO), 4-methacryloyloxy-2,2,6,6-tetramethylpiperidine-1-oxyl (TEMPO methacrylate), poly(ethylene glycol)-bis-TEMPO, polymer-bound (2,2,6,6-tetramethyl-1-piperidinyloxy (PS-TEMPO), 2,2,6,6-tetramethyl-1-piperinyloxy on silica gel, and TEMPO functionalized magnetic nano particles (TurboBeads™ TEMPO). In an even more preferred embodiment of the present invention, the organocatalyst is selected from 2,2,6,6-tetramethylpiperidin-1-oxyl (TEMPO), 4-hydroxy-2,2,6,6-tetramethylpiperidin-1-oxyl (4-hydroxyTEMPO), 4-amino-2,2,6,6-tetramethylpiperidin-1-oxyl (4-amino TEMPO), 4-carboxy-2,2,6,6-tetramethylpiperidin-1-oxyl (4-carboxy TEMPO), 4-acetamido-2,2,6,6-tetramethylpiperidine-1-oxyl (4-acetamido TEMPO), 9-azabicyclo[3.3.1]nonane-*N*-oxyl (ABNO), 9-azabicyclo[3,3,1]nonan-3-one-9-oxyl (keto-ABNO), 2,2'-azino-bis(3-ethylbenzothiazoline-6-sulphonic acid) (ABTS), VA (violuric acid), 2-azaadamantane-*N*-oxyl (AZADO), 1-methyl-2-azaadamantane-*N-*oxyl (1-methyl AZADO), 1,3-dimethyl-2-azaadamantane-*N*-oxyl (1,3-dimethyl AZADO) or *N*-hydroxyphthalimide. Yet more preferably, the organocatalyst is selected from 2,2,6,6-tetramethylpiperidin-1-oxyl, 4-hydroxy-2,2,6,6-tetramethylpiperidin-1-oxyl, 4-acetamido-2,2,6,6-tetramethylpiperidine-1-oxyl, 9-azabicyclo[3.3.1]nonane-*N*-oxyl, 2,2'-azino-bis(3-ethylbenzothiazoline-6-sulphonic acid), 2-azaadamantane-*N*-oxyl or 1-methyl-2-azaadamantane-*N*-oxyl. Even more preferably, the organocatalyst is selected from 2,2,6,6-tetramethylpiperidin-1-oxyl, 4-hydroxy-2,2,6,6-tetramethylpiperidin-1-oxyl, 2-azaadamantane-*N*-oxyl, 4-acetamido-2,2,6,6-tetramethylpiperidine-1-oxyl or 1-methyl-2-azaadamantane-*N*-oxyl.

The oxidizing agent is selected from hypohalous acids, alkali metal salts of hypohalous acids, periodinane compounds, or compositions comprising any of the aforementioned oxidizing agents. Preferably, the oxidizing agent is selected from hypochlorous acid, sodium or potassium salts of hypochlorous acid, hypobromous acid, sodium or potassium salts of hypobromous acid, iodosobenzene diacetate, phenyliodine bis(trifluoroacetate), phenyliodine diacetate, iodoxybenzene, iodylbenzene, iodosylbenzene, 2-iodoxybenzoic acid, 1,1,1-triacetoxy-1,1-dihydro-1,2-benziodoxol-3(1H)-one, or compositions comprising any of the aforementioned oxidizing agents. In one preferred embodiment of the present invention, the oxidizing agent is selected from NaClO, I₂, iodosylbenzene, 2-iodoxybenzoic acid, 1,1,1-triacetoxy-1,1-dihydro-1,2-benziodoxol-3(1 H)-one, or a mixture of NaClO, KBr and Bu₄NBr. More preferably, the oxidizing agent is selected from NaClO, I₂ or a mixture of NaClO, KBr and Bu₄NBr. Still more preferably, the oxidizing agent is selected from NaClO or a mixture of NaClO, KBr and Bu₄NBr.

In a preferred embodiment, sodium hypochlorite is added to the aqueous medium comprising co-solvent of step a) in an amount sufficient to render its concentration in said medium, prior to any oxidation taking place, more than 100 mM, more preferably 100 to 1000 mM, even more preferably 200 to 600 mM, most preferably 350 to 450 mM. In addition, the compound of formula (I) is preferably added to the aqueous medium comprising co-solvent of step (a) in an amount sufficient to render its concentration in said medium, prior to any oxidation taking place, more than 50 mM, more preferably 50 to 500 mM, even more preferably 100 to 400 mM, most preferably 250 to 350 mM in an amount of 1 to 2 equivalents.

Step (a) is performed in an aqueous medium comprising an organic co-solvent. In other words, step (a) is performed in a mixture of water and an organic co-solvent. The mixture may be biphasic or consist of one phase. Preferably the co-solvent of step (a) is selected from non-polar and polar aprotic solvents. More preferably, the co-solvent is a solvent selected from hexane, heptane, cyclohexane, ethyl acetate, *tert*-butylmethyl ether, cyclopentylmethyl ether, diethyl ether, *N,N-*dimethylformamide, tetrahydrofuran, 2-methyltetrahydrofuran, toluene, dioxane, benzene, chlorobenzene, trifluorotoluene, dichloromethane, chloroform, acetonitrile, dimethylacetamide, *N-*methylpyrrolidine or dimethyl sulfoxide. In one particularly preferred embodiment of the present invention, the co-solvent of step (a) is selected from ethyl acetate, *tert*-butylmethyl ether, cyclopentylmethyl ether, *N,N-*dimethylformamide, tetrahydrofuran, 2-methyltetrahydrofuran, toluene, trifluorotoluene, dichloromethane or chloroform. Even more preferably, the solvent is selected from toluene, trifluorotoluene, cyclopentylmethyl ether, 2-methyl tetrahydrofuran or dichloromethane. Furthermore preferably, the co-solvent of step (a) is trifluorotoluene.

Step (a) is preferably performed using a buffer solution as the aqueous medium, more preferably a basic buffer comprising an aqueous solution of a weak base and its conjugate acid with a pH greater than 7 which reduces the change of pH upon addition of small amounts of acid or base, or upon dilution. Preferably, the basic buffer comprises an aqueous solution of sodium or potassium bicarbonate, sodium or potassium acetate, sodium or potassium citrate, sodium or potassium phosphate, sodium or potassium tetraborate or organic amines selected from methylamine, isopropylamine, dimethylamine, triethylamine, diisopropylamine, pyridine, 2,6-lutidene, tetramethylguanidine, 1,8-diazabicyclo[5.4.0]undec-7-ene, more preferably sodium tetraborate or ethanolamine. The buffer is preferably selected so that step (a) is performed at a pH between 7 and 11. In one preferred embodiment of the present invention, step (a) is performed at a pH between 7 and 10. More preferably, step (a) is performed at a pH between 7.5 and 9.5, most preferably 8.5 to 9.5.

In a furthermore preferred embodiment of the present invention, step (a) is performed by adding an organocatalyst to a compound of formula (I) in an aqueous medium comprising an oxidizing agent and an organic co-solvent at a pH of between 7.5 and 9.5, wherein: the organocatalyst is selected from 2,2,6,6-tetramethylpiperidin-1-oxyl, 4-hydroxy-2,2,6,6-tetramethylpiperidin-1-oxyl or 4-acetamido-2,2,6,6-tetramethylpiperidine-1-oxyl; the oxidizing agent is selected from NaClO or a mixture of NaClO, KBr and Bu₄NBr; the co-solvent is selected from tetrahydrofuran, toluene, trifluorotoluene, dichloromethane or chloroform; and sodium hypochlorite and the compound of formula (I) are added to the aqueous medium comprising co-solvent of step a) in amounts sufficient to render their concentration in said medium, prior to any reaction taking place, 100 to 1000 mM and 50 to 500 mM, respectively.

More preferably, step (a) of the present invention is performed by adding an organocatalyst to a compound of formula (I) in an aqueous medium comprising an oxidizing agent and an organic co-solvent at a pH of between 8.5 and 9.5, wherein the organocatalyst is selected from 2,2,6,6-tetramethylpiperidin-1-oxyl, 4-hydroxy-2,2,6,6-tetramethylpiperidin-1-oxyl or 4-acetamido-2,2,6,6-tetramethylpiperidine-1-oxyl, the oxidizing agent is NaClO, the co-solvent is trifluorotoluene, and sodium hypochlorite and the compound of formula (I) are added to the aqueous medium comprising co-solvent of step a) in amounts sufficient to render their concentration in said medium, prior to any reaction taking place, 200 to 600 mM and 100 to 400 mM, respectively.

In a most preferred embodiment, the oxidation of the compound of formula (III) of step (a) is carried out in an aqueous 0.35 to 0.45 M solution of sodium hypochlorite (1 to 1.5 equivalents) at pH 8.5 to 9.5 in the presence of 0.5 to 1.5 mol% of AZADO, with trifluorotoluene as co-solvent.

Step (b) comprises converting the compound of formula (II) generated in step (b) into the compound of formula (III) by transferring the amine moiety from an amine donor compound by a transamination catalyst.

In one preferred embodiment of the present invention, the transamination catalyst is a transaminase enzyme. Said transamination catalyst has transaminase activity, wherein the term "transaminase activity" refers to an activity capable of catalyzing the conversion of a carbonyl moiety to an amine moiety (*i.e.* converting an oxy moiety into an amino moiety). Thus, a transaminase enzyme refers to a polypeptide capable of enzymatically transferring an amino group (NH₂), a pair of electrons and a proton from a primary amine to a carbonyl group (C=O) of an acceptor molecule.

The transaminase enzyme may be a purified enzyme that has been subjected to an isolation or purification procedure in order to provide it in higher purity than transaminases are found in cells. Alternatively, the transaminase enzyme may be comprised in an extract from a cell, comprised in contents of a lysed cell, bound to another biological macromolecule such as a cell membrane, protein or cofactor, present in a whole-cell sample or located in or on a cell, whereby the transaminase enzyme may be inside the cell (*i.e.* in the cytosol) or be attached to a cellular membrane in contact with the cytosol or attached to a cellular membrane in contact with the environment external to the cell. The transaminase enzyme, regardless of whether it is isolated from a cell, purified from a cell, comprised in an extract from a cell, comprised in contents of a lysed cell, bound to another biological macromolecule such as a cell membrane, protein or cofactor, present in a whole-cell sample or located in or on a cell, may be immobilized or non-immobilized. Preferably, said transaminase enzyme is present in an aqueous solution, in particular a buffered and/or saline aqueous solution.

Preferably, the transaminase enzyme is an omega-transaminase (ω-TA) enzyme which is a naturally occurring (wild type) transaminase or a non-naturally occurring engineered polypeptide generated by human intervention. In one more preferable embodiment, the transaminase enzyme is selected from the commercially available Codex^{®}Transaminase Screening Kit, even more preferably from any of ATA-007, ATA-013, ATA-024, ATA-025, ATA-033, ATA-113, ATA-117, ATA-200, ATA-217, ATA-234, ATA-237, ATA-238, ATA-251, ATA-254, ATA-256, ATA-260, ATA-301, ATA-303, ATA-412, ATA-415, ATA-P1-A06, ATA-P1-B04, ATA-P1-F03, ATA-P1-G05, ATA-P1-G06, ATA-P2-A01, ATA-P2-A07, or ATA-P2-B01 comprised therein, furthermore preferably from a transaminase enzyme comprising any of the naturally occurring or engineered amino acid sequences disclosed in US8470564, US9353355, US8889380, US8293507, US8921079, US9133445, US9434968, US9029106, US9550982, US9512410 and/or US9617573, even more preferably a transaminase enzyme comprising any amino acid sequence corresponding to the sequence of:
(a) SEQ ID NO: 2 (corresponding to a transaminase from *Vibrio fluvialis*) or any of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, or 198 (each corresponding to an artificial sequence of a variant of a transaminase from *Vibrio fluvialis*) disclosed in the 569 kB ASCII formatted text file "376247-034.txt" created on 8 January 2010, submitted via EFS-Web and incorporated by reference in US8470564, US9029106 and US9512410 in their entirety. Said amino acid sequences are available on the USPTO SCORE database via PAIR using the Supplemental Content tab provided under each of the entries of the aforementioned US patents;
(b) SEQ ID NO: 2 (corresponding to ATA-117) or any of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166 or 168 (each corresponding to an artificial sequence of a variant of ATA-117) disclosed in the 367 kB ASCII formatted text file "376247-035.txt" created on 26 February 2010, submitted via EFS -Web and incorporated by reference in US9353355, US8889380, US8293507, US9133445 and US9550982 in their entirety. Said amino acid sequences are available on the USPTO SCORE database via PAIR using the Supplemental Content tab provided under each of the entries of the aforementioned US patents, and are identical to the corresponding sequences disclosed in the 216 kB ASCII formatted text file "376247-035USP1.txt" created on 26 February 2009, which was submitted via EFS-Web and incorporated by reference in US61/155,902 in its entirety;
(c) SEQ ID NO: 2 (corresponding to ATA-117) or any of SEQ ID NO: 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166 or 168 (each corresponding to an artificial sequence of a variant of ATA-117) disclosed in the 359 kB ASCII formatted text file "CX2-019WO1_ST25.txt" created on 21 June 2010, submitted via EFS -Web and incorporated by reference in US8921079 and US9434968 in their entirety. Said amino acid sequences are available on the USPTO SCORE database via PAIR using the Supplemental Content tab provided under each of the entries of the aforementioned US patents, and are identical to the corresponding sequences disclosed in the 367 kB ASCII formatted text file "376247-042USP1.txt" created on 26 February 2010, which was submitted via EFS-Web and incorporated by reference in US61/308,873 in its entirety; or
(d) SEQ ID NO: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304 or 306 (each corresponding to an engineered transaminase variant) disclosed in the 648 kB ASCII formatted text file "CX2-129WO2_ST25.txt" created on 29 January 2014, submitted via EFS-Web and incorporated by reference in US9617573 in their entirety, which are available on the USPTO SCORE database via PAIR using the Supplemental Content tab provided under the entry of the aforementioned US patent. Alternatively, in another more preferable embodiment, the transaminase enzyme is selected from ω-TAs overexpressed in *E. coli,* even more preferably either of the (*S*)-selective transaminases from *Chromobacterium violaceum* (Cv, Enzyme Microb. Technol. 2007, 41, 628-637) or (*S*)-*Arthrobacter* (ArS, Cambrex North Brunswick Inc. WO 2006/063336A2), or either of the (*R*)-selective transaminases from (*R*)-*Arthrobacter* (ArR, Adv.Synth. Catal. 2011, 353, 3227-3233) or its evolved variant ArRmut11 (Science 2010, 329, 305-309).

Step (b) is performed in the presence of an amino donor compound. An amino donor (or amine donor) refers to an amino compound which donates an amino group to the amino acceptor, thereby itself becoming a carbonyl compound. In one preferred embodiment of the present invention, the amino donor compound comprises a primary amino moiety. Preferably, the primary amine is of formula R*NH₂ where R* is an R^{3a} substituent, as defined herein, and is unsubstituted or substituted with one or more enzymatically non-inhibiting R^{3a} or R^{3b} substituents, as defined herein, or a salt thereof, as well as any racemate, (*R*)-enantiomer or (*S*)-enantiomer thereof. More preferably, R* is selected from C₁-C₆ alkyl, C₁-C₆ alkoxy, C₁-C₆ acyl, C₁-C₆ carbamoyl, an alkylaryl group or a C₆ aryl group, and is unsubstituted or substituted with one or more enzymatically non-inhibiting R^{3a} or R^{3b} substituents. Even more preferably, the amino donor is a molecule of the general formula (IV): 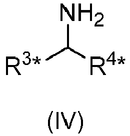 in which R^{3*} and R^{4*} are each independently selected from a C₁-C₆ alkyl, C₁-C₆ alkylaryl group, or aryl group which is unsubstituted or substituted with one or more enzymatically non-inhibiting groups, wherein R^{3*} can be the same or different from R⁴ in structure or chirality. Alternatively, R^{3*} and R^{4*}, when taken with the carbon atom to which they are both attached, complete a 5-membered to 8-membered ring of a carbocycle or a heterocycle comprising at least one heteroatom selected from O, N or S, wherein said carbocycle or heterocycle comprises one ring or two to four fused 5-membered to 8-membered rings, and wherein said carbocycle or heterocycle is unsubstituted or substituted with 1 to 4 enzymatically non-inhibiting R^{3a} or R^{3b} substituents, as defined herein. Furthermore preferably, the amino donor compound is a chiral or achiral (preferably racemic) C₁-C₆ alkyl amine, C₆ aryl amine, C₆ aryl-C₁-C₆ alkyl amine, C₁-C₆ alkyl-C₆ aryl amine, amino acid or amino-C₁-C₆ alkyl alcohol. Yet more preferably, the amino donor compound is selected from isopropylamine (also referred to as 2-aminopropane or IPM), methylamine, ethylamine, α-phenethylamine (also referred to as 1-phenylethanamine), or the racemate (*S*)(*R*)-1-phenylethanamine or enantiomers (*S*)-1-phenylethanamine or (*R*)-1-phenylethanamine thereof, 2-amino-4-phenylbutane, glycine, L-glutamic acid, L-glutamate, monosodium glutamate, L-alanine, D-alanine, D,L-alanine, L-aspartic acid, L-lysine, D,L-ornithine, β-alanine, taurine, n-octylamine, cyclohexylamine, 1,4-butanediamine, 1,6-hexanediamine, 6-aminohexanoic acid, 4-aminobutyric acid, tyramine, benzyl amine, 2-aminoethanol, 2-aminobutane, 2-amino-1-butanol, 1-amino-1-phenylethane, 1-amino-1-(2-methoxy-5-fluorophenyl)ethane, 1-amino-1-phenylpropane, hydroxyphenylpropane, 1-amino-1-(4-bromophenyl)propane, nitrophenyl)propane, 1-phenyl-2-aminopropane, 1-(3-trifluoromethylphenyl)-2-aminopropane, 2-aminopropanol, 1-amino-1-phenylbutane, 1-phenyl-2-aminobutane, 1-(2,5-dimethoxy-4-methylphenyl)-2-aminobutane, 1-phenyl-3-aminobutane, 1-(4-hydroxyphenyl)-3-aminobutane, 1-amino-2-methylcyclopentane, 1-amino-3-methylcyclopentane, 1-amino-2-methylcyclohexane, 1-amino-1-(2-naphthyl)ethane, 3-methylcyclopentylamine, 2-methylcyclopentylamine, 2-ethylcyclopentylamine, 2-methylcyclohexylamine, 3-methylcyclohexylamine, 1-aminotetralin, 2-aminotetralin, 2-amino-5-methoxytetralin or 1-aminoindan, or a salt thereof, as well as any racemate, (*R*)-enantiomer or (*S*)-enantiomer thereof. Even more preferably, the amino donor compound is selected from *iso*propylamine, benzyl amine, (*R*)-1-phenylethylamine, (*S*)-1-phenylethylamine, (*R*)(*S*)-1-phenylethylamine, L-alanine, D-alanine, D,L-alanine or 2-aminoethanol. In a particularly preferred embodiment of the present invention, an amino donor compound is selected from isopropylamine or 2-aminoethanol. In an even more preferred embodiment of the present invention the amino donor compound is isopropylamine.

Step (b) is also performed in the presence of a cofactor. A cofactor is a non-protein compound that operates in combination with an enzyme in catalyzing a reaction. Said cofactor is preferably selected from a compound of the vitamin B6 family. In one preferred embodiment of the present invention, said cofactor is selected from pyridoxal-5'-phosphate (also known as 1-(4'-formyl-3'-hydroxy-2'-methyl-5'-pyridyl)methoxyphosphonic acid, pyridoxal-phosphate, PLP, PYP, P5P or CAS number [54-47-7]), pyridoxine (PN), pyridoxal (PL), pyridoxamine (PM), pyridoxine phosphate (PNP) or pyridoxamine phosphate (PMP). Most preferably, the co-factor is pyridoxal-5'-phosphate (PLP). Pyridoxal-5'-phosphate can be produced *in vivo* by phosphorylation and oxidation of pyridoxol (also known as Vitamin B6). In transamination reactions using transaminase enzymes or polypeptides, the amine group of the amino donor is transferred to the cofactor to produce a keto byproduct, while pyridoxal-5'-phosphate is converted to pyridoxamine phosphate. Pyridoxal-5'-phosphate is regenerated by reaction with a different keto compound (the amino acceptor). The transfer of the amine group from pyridoxamine phosphate to the amino acceptor produces a chiral amine and regenerates the cofactor.

Step (b) is performed by adding an aqueous buffer to the mixture obtained in step (a), wherein the transamination catalyst is added at the same time as said aqueous buffer or after adding said aqueous buffer. Preferably the transamination catalyst is added after adding said aqueous buffer to the mixture obtained in step (a). It is essential that the transamination catalyst is not added to the mixture obtained in step (a) without adding said buffer in order to ensure effective transamination.

The aqueous buffer added in step (b) is preferably a basic buffer comprising an aqueous solution of a weak base and its conjugate acid with a pH greater than 7 which reduces the change of pH upon addition of small amounts of acid or base, or upon dilution. Preferably, the basic buffer comprises an aqueous solution of sodium or potassium bicarbonate, sodium or potassium carbonate, sodium or potassium acetate, sodium or potassium citrate, sodium or potassium phosphate, sodium or potassium tetraborate or organic amines selected from methylamine, isopropylamine, dimethylamine, triethylamine, diisopropylamine, pyridine, 2,6-lutidene, tetramethylguanidine, 1,8-diazabicyclo[5.4.0]undec-7-ene, triethanolamine or tris(hydroxymethyl)aminomethane. More preferably the basic buffer comprises an aqueous solution of potassium phosphate, potassium carbonate, potassium bicarbonate, sodium tetraborate, triethanolamine, tris(hydroxymethyl)aminomethane or ethanolamine. Even more preferably the basic buffer comprises an aqueous solution of potassium phosphate. The buffer is preferably selected so that step (b) is performed at a pH between 7 and 12. In one embodiment, step (b) is performed by adding the transamination catalyst after adding the aqueous buffer to the mixture obtained in step (a), wherein said buffer has a pH between 7.5 and 12. More preferably, said buffer has a pH between 7.5 and 11.5. Even more preferably, said buffer has a pH between 7.5 and 9.5 or a pH between 9.5 and 11.5.

The aqueous buffer added in step (b) preferably dilutes the mixture obtained in step (a) by at least 1.5 times. In one more preferred embodiment of the present invention, the aqueous buffer added in step (b) dilutes the mixture obtained in step (a) by at least 2 times [*i.e.* two-fold, so the concentration of the mixture obtained in step (a) is at most halved]. Even more preferably, the aqueous buffer added in step (b) preferably dilutes the mixture obtained in step (a) by at least 5 times (*i.e.* five-fold), yet more preferably at least 10 times (*i.e.* ten-fold), and most preferably from 10 to 100 times (*i.e.* ten to 100-fold).

Preferably, step (b) additionally comprises adding a co-solvent selected from any water soluble or water miscible non-polar, polar aprotic or polar protic solvent to the mixture obtained in step (a). More preferably, said co-solvent is a solvent selected from dimethyl sulfoxide (DMSO), dimethyl acetamide, dimethyl formamide (DMF), acetonitrile (MeCN), tetrahydrofuran (THF), *N-*methylpyrrolidine, an alcohol, methoxyethanol, ethoxyethanol, ethylene glycol or an ether of ethylene glycol. In one embodiment, step (b) additionally comprises adding a co-solvent selected from dimethyl sulfoxide, dimethyl acetamide, dimethyl formamide, acetonitrile, tetrahydrofuran, methanol, ethanol, isopropanol, butanol, sec-butanol or trifluoroethanol, ethylene glycol or an ether of ethylene glycol to the mixture obtained in step (a). In one particularly preferred embodiment, step (b) additionally comprises adding a co-solvent selected from solvent is selected from DMSO, methanol, ethanol, isopropanol, butanol, sec-butanol or trifluoroethanol, ethylene glycol or an ether of ethylene glycol to the mixture obtained in step (a).

In a furthermore preferred embodiment, step (b) is performed by adding an aqueous phosphate buffer, an omega-transaminase enzyme, an amine donor compound and a cofactor to the mixture obtained in step (a), wherein said omega-transaminase enzyme is added after adding said buffer having a pH between 7.5 and 11.5, wherein the cofactor is pyridoxal-5'-phosphate, and the amine donor compound is isopropylamine, and said buffer and isopropylamine are added to the mixture obtained in step a) in amounts sufficient to render their concentration in said mixture, prior to any reaction taking place, 50 to 500 mM and 0.2 to 10 M, respectively.

In an alternative furthermore preferred embodiment, step (b) is performed by adding an aqueous buffer comprising Tris-HCl in a concentration of 100 mM, an omega-transaminase enzyme, an amine donor compound and a cofactor to the mixture obtained in step (a), wherein said omega-transaminase enzyme is added after adding said buffer having a pH between 9 and 12, wherein the cofactor is pyridoxal-5'-phosphate, and the amine donor compound is isopropylamine, and said buffer and *iso*propylamine are added to the mixture obtained in step a) in amounts sufficient to render their concentration in said mixture, prior to any reaction taking place, 50 to 500 mM and 0.2 to 10 M, respectively.

The method of the present invention is a one-pot sequential (also known as a cascade, domino, tandem, stepwise, concurrent, one-pot two steps) reaction since step (b) is performed upon the mixture obtained according to step (a). In other words, the transamination reaction is performed on the compound of formula (II) which is obtained from the oxidation of the compound of formula (I) that is effected by forming the mixture according to step (a). Importantly, the one-pot method of the present invention involves sequential steps [as opposed to a one-pot concurrent reaction where steps (a) and (b) are performed concurrently], wherein further reaction according to step (a) does not occur once step (b) has been initiated. This means that the present invention involves successive reactions of steps (a) and (b) in one pot and does not involve the isolation or purification of the compound of formula (II), nor the removal of the aqueous medium comprising a co-solvent used in step (a) prior to performing the transamination reaction. Thus, the present invention provides a method that may be used to produce amines starting from compounds comprising a hydroxyl group, preferably attached to an aromatic or aliphatic hydrocarbon, without the need for employing procedures that involve purifying intermediates, extraction or a change of solvent.

As used herein, the phrase "isolation" or "purification" refers to techniques known in the art by which one may obtain the compound of formula (III). Examples of such techniques include, but are not limited to chromatographic techniques, crystallization, filtration or distillation, *etc.*

A variety of chromatographic techniques may be employed in the obtention of Formula (III). Chromatographic techniques used in the method of the present invention preferably are selected from High Performance Liquid Chromatography (HPLC) including normal-, reversed- or chiral-phase HPLC; Medium Pressure Liquid Chromatography (MPLC), Super Critical Fluid Chromatography; preparative Thin Layer Chromatography (prep TLC); Gas Chromatography (GC); flash chromatography with silica gel or reversed-phase silica gel; ion-exchange chromatography; or radial chromatography.

Abbreviations that are used herein include Ac (acyl [CH₃C(O)-]), Aq. (aqueous), AZADO (2-azaadamantane-N-Oxyl), Bn (benzyl), BOC (*tert-*butyloxycarbonyl), °C (degrees Celsius), c (conversion), calc. (calculated), DMAc (*N,N-*dimethylacetamide), Cbz (benzyloxycarbonyl), DCM (dichloromethane), DKR (dynamic kinetic resolution), DIPEA (*N,N*-di*i*sopropylethylamine), DMAc (*N,N*-dimethylacetamide), DMF (*N,N*-dimethylformamide), DMSO (dimethyl sulfoxide), equiv. [equivalent(s)], Et (ethyl), EtOAc (ethyl acetate), h [hour(s)], HMDS (hexamethyldisilazane), HPLC (high performance liquid chromatography), IPA (isopropyl alcohol), IPAC (isopropyl acetate), IPM (isopropylamine), ACN (acetonitrile), mp (melting point), MS (mass spectrum), MTBE (methyl *tert-*butyl ether), NaOCl (sodium hypochlorite), PG (protecting group), Ph (phenyl), Phth (phthaloyl), Prep. (preparative), r.t. [(or rt or RT) room temperature, between 20 and 25 °C, typically 22 °C], TEA (triethyl amine), TFA (trifluoroacetic acid), THF (tetrahydrofuran) and TLC (thin layer chromatography).

In the present disclosure, the technical and scientific terms used in the descriptions herein will have the meanings commonly understood by one of ordinary skill in the art, unless specifically defined otherwise. Accordingly, the following terms are intended to have the following meanings:
"protein", "polypeptide" and "peptide" are used interchangeably herein to denote a polymer of at least two amino acids covalently linked by an amide bond, regardless of length or post-translational modification (*e.g.* glycosylation, phosphorylation, lipidation, myristylation or ubiquitination, *etc*.). Included within this definition are proteins of D- and/or L-amino acids;
"acid" or "acidic aqueous solution" refers to an aqueous solution with a pH less than 7. Examples of an acidic aqueous solution are aqueous solutions of hydrochloric acid, hydrobromic acid, sulfuric acid, acetic acid, glycolic acid or citric acid, *etc*.;
"conversion" refers to the enzymatic conversion of the substrate(s) of the formula (I) and/or (II) to the corresponding product(s) of the formula (III). Thus, "percent conversion" refers to the percent of the compound of formula (I) or the percent of the compound of formula (II) that is converted to the compound of formula (III). Thus, in the case of the percent conversion of the compound of formula (II) into the compound of formula (III), this relates to the "enzymatic activity" or "activity" of a transaminase polypeptide expressed as a "percent" of the substrate of formula (II) to the product of formula (III);
"alkyl" refers to a radical of a saturated linear hydrocarbon consisting of carbon and hydrogen atoms, preferably a C₁-C₁₂ alkyl, more preferably a C₁-C₈ alkyl. For example, "alkyl" may be methyl, ethyl, *n*-propyl, *i*-propyl, *n*-butyl, *t*-butyl, *n*-pentyl, *n*-hexyl, *n*-heptyl or *n*-octyl, *etc.* Alkyl radicals may be optionally substituted by one or more substituents, preferably 1 to 5, more preferably 1 or 2, such as halogen, hydroxyl, alkoxy such as *O*-propyl or *O*-benzyl, benzoate, carboxyl, cyano, carbonyl, acyl, alkoxycarbonyl, amino, imino, nitro, mercapto and alkylthio;
"alkenyl" refers to a radical of a hydrocarbon chain consisting of carbon and hydrogen atoms, comprising at least one carbon-carbon (C=C) double bond, preferably a C₂-C₁₂ alkenyl, more preferably a C₂-C₈ alkenyl. For example, "alkenyl" may be ethenyl, *n*-propenyl, *i*-propenyl, prop-2-enyl (allyl), *n*-butenyl or *n*-pentenyl, *etc.* Alkenyl radicals may be optionally substituted by one or more substituents such as halogen, hydroxyl, alkoxy such as *O*-propyl or *O*-benzyl, benzoate, carboxyl, cyano, carbonyl, acyl, alkoxycarbonyl, amino, imino, nitro, mercapto and alkylthio;
"alkynyl" refers to a radical of a hydrocarbon chain consisting of carbon and hydrogen atoms, comprising between one and three carbon-carbon (C=C) triple bonds, preferably a C₂-C₁₂ alkynyl, more preferably a C₂-C₈ alkynyl. For example, "alkynyl" may be -C=CH, -CH₂C≡CH, C≡CCH₃ or CH₂C≡CCH₃, *etc*.;
"carbocyclyl" refers to a radical of a "carbocycle" selected from a cycloalkyl, cycloalkenyl or aryl radical.
"cycloalkyl" refers to radical of an alicyclic saturated hydrocarbon (cycloalkane) comprising one ring or between two and four fused 3-membered to 8-membered rings, wherein said cycloalkyl is a 3 to 15-membered cycloalkyl, preferably a C₅-C₁₀ monocyclic cycloalkyl, more preferably a C₅-C₈ monocyclic cycloalkyl. For example, "cycloalkyl" may be cyclopentyl, cyclohexyl, cycloheptyl, cycoloctyl, dicyclopentanyl or dicyclohexanyl, *etc.* Cycloalkyl radicals may be optionally substituted with 1, 2 or 3 residues of monovalent saturated linear or branched hydrocarbon, such as cyclopropyl, cyclobutyl, cyclopentyl, cyclohexyl, cycloheptyl, methylcyclohexyl or dimethylcyclohexyl, *etc.;*
"cycloalkenyl" refers to radical of an alicyclic unsaturated hydrocarbon (cycloalkene) comprising one ring or between two and four fused 5-membered to 8-membered rings and one or two carbon-carbon (C=C) double bonds, preferably a monocyclic C₅-C₁₀ cycloalkenyl comprising one carbon-carbon double bond, more preferably a C₅-C₈ monocyclic cycloalkenyl comprising one carbon-carbon double bond. For example, "cycloalkenyl" may be cyclopentenyl, cyclohexenyl, cycloheptenyl, cyclooctyl or dicyclopentenyl *etc*.;
"aryl" refers to a radical of a hydrocarbon (arene) comprising one ring or between two and four fused 5-membered to 8-membered rings, at least one of said rings being an aromatic ring, preferably a monocyclic or bicyclic C₅-C₁₀ aryl comprising one or two aromatic rings, more preferably a monocyclic or bicyclic C₅-C₉ aryl comprising one aromatic ring. For example, "aryl" may be phenyl, naphthyl, anthracenyl, indanyl or indenyl, *etc*.;
"heterocyclyl" refers to a radical of a compound (heterocycle) comprising at least one heteroatom selected from O, N or S and one ring or two to four fused 3-membered to 8-membered rings, wherein said heterocycle is a 3 to 15-membered heterocycle, preferably a monocyclic or fused-bicyclic 5 to 10-membered heterocycle, more preferably a monocyclic or fused-bicyclic 5 to 9-membered heterocycle wherein each ring is a 5 or 6-membered ring. Said heterocycle may be saturated, unsaturated or aromatic. For example, the "heterocycle" from which the heterocyclyl radical is derived may be selected from azepine, benzimidazole, benzothiazole, isothiazole, imidazole, indole, piperidine, piperazine, purine, quinoline, thiadiazole, pyrrolidine, tetrahydrofuran, tetrahydrothiophene, pyrrole, furan, thiophene, imidazolidine, pyrazolidine, imidazole, pyrazole, oxazolidine, isoxazolidine, oxazole, isoxazole, thiazolidine, isothiazolidine, thiazole, dioxolane, dithiolane, pyridine, tetrahydropyran, morpholine, isoindole, isoindoline, quinoline, isoquinoline, benzazepine, carbazole or acridine, *etc.* When the heterocyclyl moiety is substituted, substitution is at one or more atoms of nitrogen, carbon or sulfur and the nitrogen atom can be optionally quaternized;
"hydroxyalkyl" refers to an alkyl residue as defined above, substituted with 1, 2 or 3 hydroxy groups. For example, "hydroxyalkyl" may be -CH₂OH, -CH₂CH₂OH, -CH(OH)CH₃, -CH₂CH₂CH₂OH or -CH(OH)CH(OH)CH₂OH, *etc*.;
"halogenated alkyl" or "haloalkyl" refers to an alkyl residue as defined above, substituted with at least one halogen, *i.e.* where any given hydrogen atom of said alkyl residue is replaced with a substituent selected from the group consisting of -F, -Cl, -Br and -I. Haloalkyl groups may be substituted at each carbon by the same or a different halogen. For example, if all hydrogen atoms of the alkyl are replaced by halogen groups it is defined as "perhalogenated" alkyl, if all hydrogen atoms of the alkyl are substituted by fluorine atoms it is defined "perfluorinated" alkyl, or if all hydrogen atoms of the alkyl are replaced by chlorine atoms it is defined as "perchlorated" alkyl. Alternatively, some but not all hydrogen atoms of the alkyl may be replaced with a halogen, for example, 1, 2, 3 or more positions. Non-limiting examples of haloalkyl groups are CH₂F, -CH₂Cl, -CH₂Br, -CHF₂, -CF₃, -CCl₃ or -CF₂CF₃, *etc*.;
"alkoxy" refers to a radical of the formula -O-alkyl, where alkyl is defined above, for example, methoxy, ethoxy, propoxy, *etc*.;
"arylalkyl" or "aralkyl" refers to an aryl group attached to an alkyl group such as benzyl, phenethyl or fluorenylethenyl; and
"silyl" refers to a -Si(R^{3a})₃ radical, preferably a trialkylsilyl group wherein the silicon is substituted by three alkyl groups, wherein each alkyl group is independently selected from the aforementioned definition of "alkyl", as defined above. For example, "silyl" may be trimethylsilyl (TMS), triethylsilyl, *tert*-butyldimethylsilyl ("TBDMS"), *tert*-butyldiphenylsilyl, tri-isopropylsilyl, diethylisopropylsilyl, texyldimethylsilyl ether, triphenylsilyl or di-*tert*-butylmethylsilyl, *etc*.;

Bearing this in mind, in the present invention:
(i) R¹ is an R³ substituent and R² is an R³ substituent, wherein each R³ substituent is independently selected from:
   - an R^{3a} substituent;
   - an R^{3b} substituent; or
   - an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with
      - an R^{3a} substituent,
      - an R^{3b} substituent, or
      - an R^{3a} substituent that is optionally substituted with an R^{3b} substituent,
   or
(ii) R¹ and R², when taken with the carbon atom to which they are both attached, complete a non-aromatic 5-membered to 8-membered ring of a carbocycle or a heterocycle comprising at least one heteroatom selected from O, N or S, wherein said carbocycle or heterocycle comprises one ring or two to four fused 5-membered to 8-membered rings, and wherein said carbocycle or heterocycle is unsubstituted or substituted at one or more atoms of N, C or S with 1 to 4 R³ substituents, wherein each R³ substituent is independently selected from:
   - an R^{3a} substituent;
   - an R^{3b} substituent;
   - an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with an R^{3b} substituent;
   - =O;
   - =NR⁴; or
   - =S,
   and wherein:
   - each R^{3a} substituent is independently selected from an alkyl, an alkenyl, an alkynyl, a carbocyclyl or a heterocyclyl moiety comprising at least one heteroatom selected from O, N or S, wherein said carbocyclyl or a heterocyclyl moiety comprises one to four fused 5-membered to 8-membered rings;
   - each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN,-Si(R⁵)₃, -SR⁵, -(S=O)-R⁵, -(S=NR⁴)-R⁵, -S(=O)₂-R⁵, -S(=O)₂-NR⁴R⁶ or -S(=O)(=NR⁴)-R^{3a};
   - R⁴ is H or R⁶;
   - R⁵ is H or an R^{3a} substituent that is optionally substituted with another R^{3a} substituent;
   - R⁶ is an R^{3a} substituent or -(C=O)-OR⁵; and
   - Z is halogen.

Preferably,
(i) R¹ is an R³ substituent and R² is an R³ substituent, wherein each R³ substituent is independently selected from:
   - an R^{3a} substituent;
   - an R^{3b} substituent; or
   - an R^{3a} substituent which is substituted with 1 to 4 substituents each independently selected from an R^{3b} substituent or an R^{3a} substituent that is optionally substituted with
      - an R^{3a} substituent,
      - an R^{3b} substituent, or
      - an R^{3a} substituent that is optionally substituted with Z or an R^{3b} substituent,
   and wherein
   - each R^{3a} substituent is independently selected from a C₁-C₁₂ alkyl or a carbocyclyl moiety wherein said carbocyclyl moiety is a monocyclic or bicyclic C₅-C₉ aryl comprising one aromatic ring;
   - each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN, or-Si(R^{3a})₃,
   - R⁴ is H or R⁶;
   - R⁵ is H or an R^{3a} substituent that is optionally substituted with another R^{3a} substituent;
   - R⁶ is an R^{3a} substituent or -(C=O)-OR⁵; and
   - Z is halogen.
   or
(ii) R¹ and R², when taken with the carbon atom to which they are both attached, complete a non-aromatic 5-membered to 8-membered ring of a carbocycle or a heterocycle comprising at least one N, O or S atom, wherein said carbocycle or heterocycle comprises one ring or two fused 5-membered to 8-membered rings, and wherein said carbocycle or heterocycle is unsubstituted or substituted with 1 or 2 R³ substituents, wherein each R³ substituent is independently selected from:
   - an R^{3a} substituent;
   - an R^{3b} substituent;
   - an R^{3a} substituent which is substituted with 1 to 4 substituents each independently selected from =O, an R^{3b} substituent or an R^{3a} substituent that is optionally substituted with an R^{3b} substituent; or
   - =O;
and wherein:
- each R^{3a} substituent is independently selected from a C₁-C₁₂ alkyl, a carbocyclyl or a heterocyclyl moiety comprising at least one N atom, wherein said carbocyclyl or a heterocyclyl moiety comprises one 5-membered to 8-membered ring or two fused 5-membered to 8-membered rings;
- each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN or-Si(R^{3a})₃;
- R⁴ is H or R⁶;
- R⁵ is H or an R^{3a} substituent that is optionally substituted with another R^{3a} substituent; and
- R⁶ is an R^{3a} substituent or -(C=O)-OR⁵.

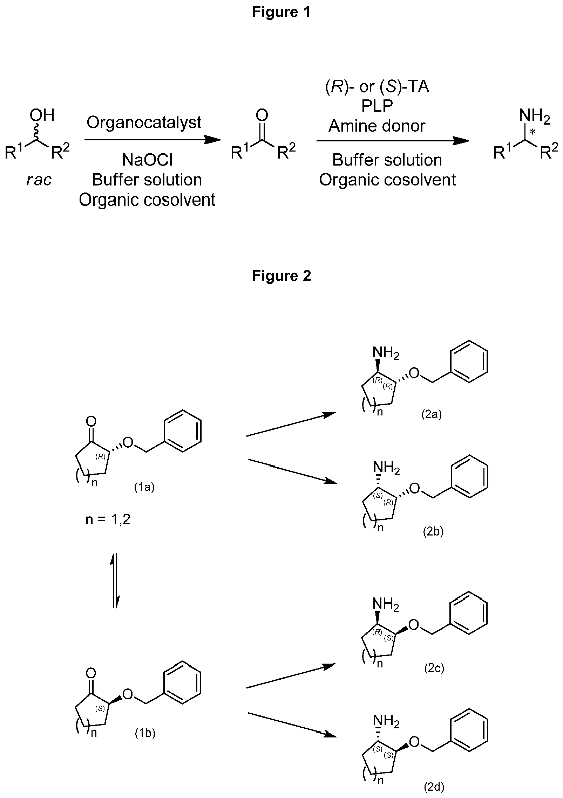

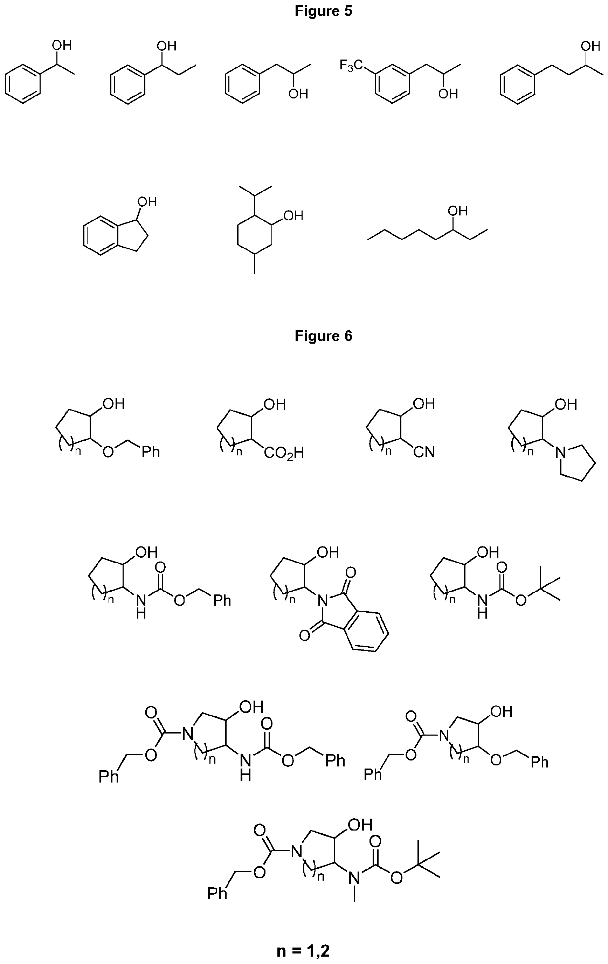

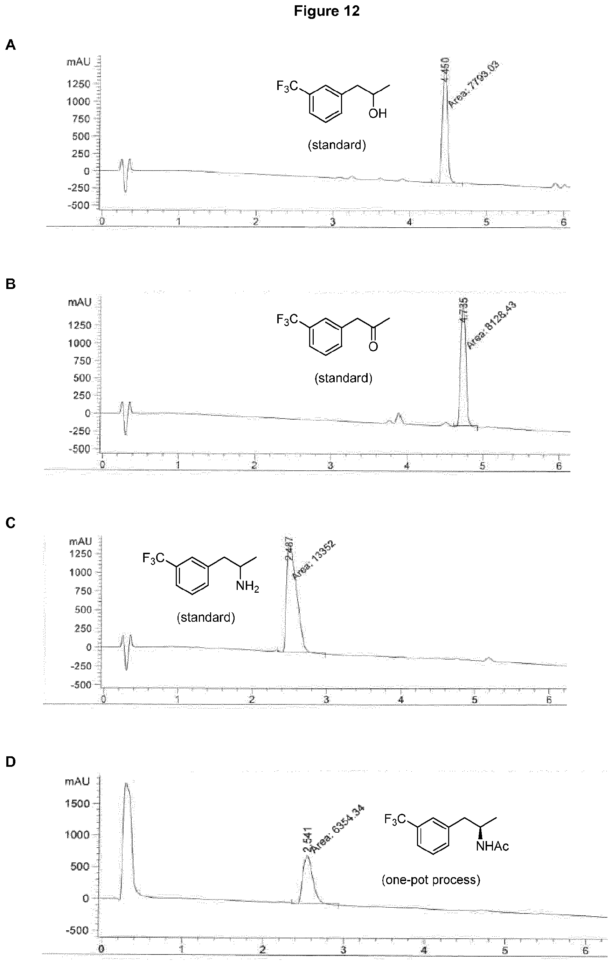

In an even more preferred embodiment of the present invention, the compound of formula (I) is selected from:
(i) an alcohol disclosed in Figure 5, Figure 6, Figure 8, Figure 12A, Figure 13A, Table 1 or Table 9; or
(ii) an alcohol from which a ketone disclosed in Figure 2, Figure 3, Figure 12B, Figure 13B or Table 1 to Table 8 is obtained according to step (a) of the method of the present invention,
or a salt thereof. Accordingly, the compound of formula (II) is selected from:
(iii) a ketone disclosed in Figure 2, Figure 3, Figure 12B, Figure 13B or Table 1 to Table 8; or
(ii) a ketone obtained from an alcohol disclosed in Figure 5, Figure 6, Figure 8, Figure 12A, Figure 13A, Table 1 or Table 9 is obtained according to step (a) of the method of the present invention,
or a salt thereof.

In one still more preferred special embodiment of the present invention, R¹ is an R³ substituent and R² is an R³ substituent, wherein each R³ substituent is independently selected from:
- an R^{3a} substituent;
- an R^{3a} substituent which is substituted with an R^{3a} substituent; or
- an R^{3a} substituent which is substituted with an R^{3a} substituent that is substituted with Z,
and wherein
- each R^{3a} substituent is independently selected from a C₁-C₈ alkyl or a carbocyclyl moiety wherein said carbocyclyl moiety is a monocyclic or bicyclic C₅-C₉ aryl comprising one aromatic ring; and
- Z is F or Cl.

Furthermore preferably, R¹ is an R³ substituent and R² is an R³ substituent, wherein each R³ substituent is independently selected from:
- an R^{3a} substituent;
- an R^{3a} substituent which is substituted with an R^{3a} substituent; or
- an R^{3a} substituent which is substituted with an R^{3a} substituent that is substituted with F,
and wherein
e ach R^{3a} substituent is independently selected from a C₁-C₆ alkyl or a carbocyclyl moiety wherein said carbocyclyl moiety is a monocyclic aryl or a bicyclic C₅-C₉ aryl comprising one aromatic ring,
wherein
step (b) is carried out at a pH of between 7.5 and 11 and the compound of formula (III) is formed by dynamic kinetic resolution, and the transaminase enzyme is selected from:
(a) ATA-013, ATA-024, ATA-025, ATA-033, ATA-117, ATA-301, ATA-303, ATA-412, ATA-415, P2-A01, P2-A07, P2-B01, ArR or ArRmut11, more preferably ATA-013, ATA-024, ATA-117, ATA-301, ATA-303, ATA-412, P2-A01, P2-A07 or P2-B01 (in this way, the compound of formula (III) is enriched in the enantiomer having an *R-*amino group); or
(b) ATA-113, ATA-200, ATA-217, ATA-234, ATA-237, ATA-238, ATA-251, ATA-254, ATA-256, ATA-260, P1-A06, P1-B04, P1-F03, P1-G05, P1-G06, ArS or Cv, more preferably ATA-113, ATA-200, ATA-237, ATA-238, ATA-251, ATA-254, ATA-256, ATA-260, P1-A06, P1-B04, P1-G05, P1-G06, ArS or Cv (in this way, the compound of formula (III) is enriched in the enantiomer having an *S*-amino group).

In another still more preferred special embodiment of the present invention, the compound of formula (I) is a compound of formula (Ia), 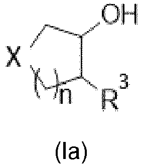 the compound of formula (II) is a compound of formula (IIa), 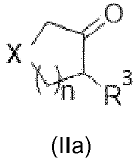 and the compound of formula (III) is a compound of formula (IIIa) 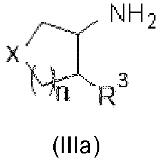 wherein
the compound of formula IIIa is formed enriched in one enantiomer and/or one diastereomer thereof,
n represents a whole number selected from 1, 2 or 3,
X is selected from CR⁵R⁵, CR⁵R⁶, O, SO₂, S, NH or NR⁴, and
(i) R³ is selected from:
   - an R^{3a} substituent;
   - an R^{3b} substituent;
   - an R^{3a} substituent which is substituted with 1 or 2 substituents each independently selected from =O, an R^{3b} substituent or an R^{3a} substituent that is optionally substituted with an R^{3b} substituent,
   or alternatively
(ii) R³, when taken with the carbon atom to which it is attached and the n carbon atom adjacent thereto [which is not the carbon atom to which the hydroxyl, oxo or amino group of formula (Ia), (IIa) or (IIIa), respectively, is bound], completes a 5-membered to 7-membered ring of a carbocycle or a heterocycle comprising at least one N atom, wherein said ring is unsubstituted or substituted with 1 or 2 substituents independently selected from:
   - an R^{3a} substituent;
   - an R^{3b} substituent;
   - an R^{3a} substituent which is substituted with 1 or 2 substituents each independently selected from =O, an R^{3b} substituent or an R^{3a} substituent that is optionally substituted with an R^{3b} substituent,
and wherein:
- each R^{3a} substituent is independently selected from C₁-C₁₂ alkyl, a carbocyclyl or a heterocyclyl moiety comprising at least one N atom, wherein said carbocyclyl or a heterocyclyl moiety comprises one 5-membered to 8-membered ring or two fused 5-membered to 8-membered rings;
- each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN or -Si(R^{3a})₃;
- each R⁴ substituent is independently selected from H or R⁶;
- each R⁵ substituent is independently selected from H or an R^{3a} substituent that is optionally substituted with another R^{3a} substituent; and
- R⁶ is an R^{3a} substituent or -(C=O)-OR⁵.

In an even more preferred embodiment of the present invention, the compound of formula (I) is a compound of formula (Ia), 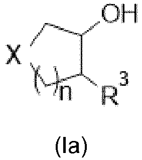 the compound of formula (II) is a compound of formula (IIa), 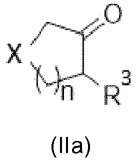 and the compound of formula (III) is a compound of formula (IIIa) 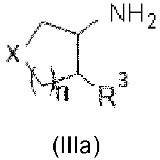 wherein
the compound of formula IIIa is formed enriched in one enantiomer and/or one diastereomer thereof,
n represents a whole number selected from 1, 2 or 3,
X is selected from CR⁵R⁵, CR⁵R⁶, O or SO₂, and
(i) R³ is selected from:
   - an R^{3a} substituent;
   - an R^{3b} substituent;
   - an R^{3a} substituent which is substituted with 1 or 2 substituents each independently selected from =O, an R^{3b} substituent or an R^{3a} substituent that is optionally substituted with an R^{3b} substituent,
   or alternatively
(ii) R³, when taken with the carbon atom to which it is attached and the n carbon atom adjacent thereto, completes a 5-membered to 7-membered ring of a carbocycle, wherein said ring is unsubstituted,
   and wherein:
   - each R^{3a} substituent is independently selected from a C₁-C₈ alkyl moiety, a carbocyclyl moiety which is a monocyclic or bicyclic C₅-C₁₀ aryl, or a heterocyclyl moiety comprising at least one N atom, wherein said heterocyclyl moiety comprises one 5-membered or 6-membered ring or two fused 5-membered or 6-membered rings;
   - each R^{3b} substituent is independently selected from -OR⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN or -Si(R^{3a})₃;
   - each R⁴ substituent is independently selected from H or R⁶;
   - each R⁵ substituent is independently selected from H or an R^{3a} substituent that is optionally substituted with another R^{3a} substituent; and
   - R⁶ is an R^{3a} substituent or -(C=O)-OR⁵.

In a still more preferred embodiment of the present invention, the compound of formula (I) is a compound of formula (Ia), 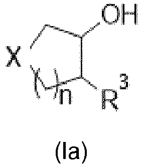 the compound of formula (II) is a compound of formula (IIa), 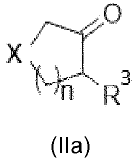 and the compound of formula (III) is a compound of formula (IIIa) 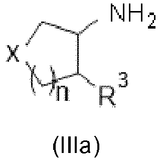 wherein step (b) is carried out at a pH of between 9 and 12 and the compound of formula (IIIa) is formed by dynamic kinetic resolution, and
n represents a whole number selected from 1, 2 or 3,
X is selected from CH₂, CHR³ or O, and
each R³ is selected from:
- an R^{3a} substituent;
- an R^{3b} substituent;
- an R^{3a} substituent which is substituted with 1 or 2 substituents each independently selected from =O, an R^{3b} substituent or an R^{3a} substituent that is optionally substituted with an R^{3b} substituent,
wherein:
- each R^{3a} substituent is independently selected from a C₁-C₈ alkyl moiety, a carbocyclyl moiety which is a phenyl, or a heterocyclyl moiety comprising at least one N atom, wherein said heterocyclyl moiety comprises one or two fused 5-membered or 6-membered rings; each R^{3b} substituent is independently selected from -OR⁵, -(C=O)-OR⁵, -CN or -NH-(C=O)-OR⁵;
- each R⁵ substituent is independently selected from H or an R^{3a} substituent that is optionally substituted with another R^{3a} substituent; and
- R⁶ is -(C=O)-OR⁵.

In one especially more preferred embodiment of the present invention, the compound of formula (III) is formed enriched in one diastereomer of:
(i) formula (IIIb1) or (IIIb2), 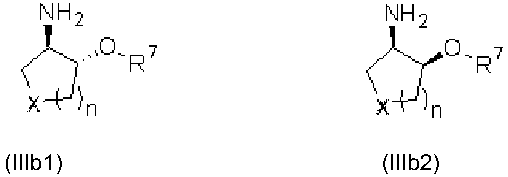 or a salt thereof; or
(ii) formula (IIIc1) or (IIIc2), 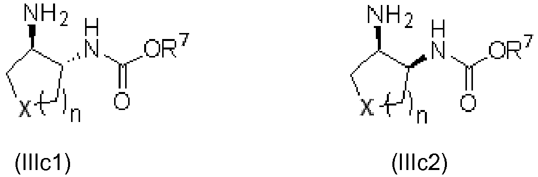 or a salt thereof,
   wherein
   n represents a whole number selected from 1, 2 or 3,
   X is selected from CH₂ or O,
   and R⁷ is selected from a benzyl or a C₁-C₈ alkyl moiety. Preferably, R⁷ is selected from a benzyl or a C₁-C₈ alkyl moiety.

In another even more preferable especial preferred embodiment of the present invention, the compound of formula (I) is a compound of formula (Ia), 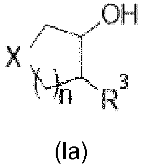 the compound of formula (II) is a compound of formula (IIa), 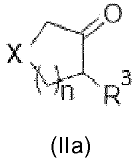 and the compound of formula (III) is a compound of formula (IIIa) 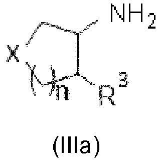 wherein
n represents a whole number selected from 1, 2 or 3,
X is selected from CH₂, CHR³ or O, and
R³ is selected from:
   - an R^{3a} substituent;
   - an R^{3b} substituent;
   - an R^{3a} substituent which is substituted with 1 or 2 substituents each independently selected from =O, an R^{3b} substituent or an R^{3a} substituent that is optionally substituted with an R^{3b} substituent,
wherein:
- each R^{3a} substituent is independently selected from a C₁-C₈ alkyl moiety, a carbocyclyl moiety which is a phenyl, or a heterocyclyl moiety comprising at least one N atom, wherein said heterocyclyl moiety comprises one or two fused 5-membered or 6-membered rings;
- each R^{3b} substituent is independently selected from -OR⁵, -(C=O)-OR⁵, -CN or -NH-(C=O)-OR⁵; and
- each R⁵ substituent is independently selected from H or an R^{3a} substituent that is optionally substituted with another R^{3a} substituent;
wherein
step (b) is carried out at a pH of between 7.5 and 11.5 and the compound of formula (IIIa) is formed by dynamic kinetic resolution, and the transaminase enzyme is selected from:
(a) ATA-007, ATA-013, ATA-024, ATA-025, ATA-033, ATA-301, ATA-303, ATA-412, ATA-415, P2-A07 or P2-B01, more preferably from P2-A07 or P2-B01 (in this way, the *cis*-isomer of formula (IIIa) is enriched in the diastereoisomer having an *R*-amino group [*i.e.* (1 R,2S)] and the *trans*-isomer of formula (IIIa) is enriched in the diastereoisomer having an *R*-amino group [*i.e.* (1*R*,2*R*)]); or
(b) ATA-113, ATA-217, ATA-234, ATA-237, ATA-238, ATA-251, ATA-254, ATA-256, ATA-260, P1-A06, P1-B04, P1-F03, P1-G05 or P1-G06, more preferably ATA-113, ATA-251, ATA-254, ATA-256, P1-A06 or P1-G06 (in this way, the *cis*-isomer of formula (IIIa) is enriched in the diastereoisomer having an *S*-amino group [*i.e.* (1*S*,2*R*)] and the *trans-*isomer of formula (IIIa) is enriched in the diastereoisomer having an *S*-amino group [*i.e.* (1*S*,2*S*)]).

Even more preferably yet, step (b) is carried out:
(i) at a pH of between 9.5 and 10.5 and the transaminase enzyme is selected from:
   (a) ATA-301 or ATA-303; or
   (b) ATA-251, ATA-254, ATA-256, ATA-260; or
(ii) at a pH of between 10.5 and 11.5 and the transaminase enzyme is selected from ATA-013, ATA-025, ATA-303, ATA-412 or ATA-415.

In a much more preferred embodiment of each of the aforementioned still more preferred special embodiments of the present invention, in step (a):
the organocatalyst is selected from 2,2,6,6-tetramethylpiperidin-1-oxyl, 4-hydroxy-2,2,6,6-tetramethylpiperidin-1-oxyl, 2-azaadamantane-*N*-oxyl, 4-acetamido-2,2,6,6-tetramethylpiperidine-1-oxyl or 1-methyl-2-azaadamantane-*N*-oxyl;
the oxidizing agent is selected from NaClO or a mixture of NaClO, KBr and Bu₄NBr;
the organic co-solvent is selected from toluene, trifluorotoluene, cyclopentylmethyl ether, 2-methyl tetrahydrofuran or dichloromethane; and
in step (b):
   the aqueous buffer is a basic buffer comprising an aqueous solution of potassium phosphate, potassium carbonate, potassium bicarbonate, sodium tetraborate, triethanolamine, tris(hydroxymethyl)aminomethane or ethanolamine, and having a pH of between 7.5 and 11.5; the transamination catalyst is an omega-transaminase;
   the amine donor compound is selected from isopropylamine, benzyl amine, (R)-1-phenylethylamine, (*S*)-1-phenylethylamine, (*R*)(*S*)-1-phenylethylamine, L-alanine, D-alanine, D,L-alanine or 2-aminoethanol; and wherein
   said transamination catalyst is added after adding said aqueous buffer, and adding the aqueous buffer to the mixture obtained in step (a) dilutes said mixture at least two-fold,

In a preferred embodiment of the present invention, the compound of the formula (III) is formed in a stereoselective manner, whereby the term "stereoselective" refers to the preferential formation of one stereoisomer over another. Stereoselectivity can be partial, where the formation of one stereoisomer is favored over another, or it may be complete where only one stereoisomer is formed.

Thus, for the purposes of the present invention, the term "highly stereoselective" refers to a transaminase polypeptide that is capable of converting the substrate to the corresponding chiral amine product with at least 85% stereoisomeric excess.

When the stereoisomers are enantiomers, the stereoselectivity is referred to as enantioselectivity, the fraction (typically reported as a percentage) of one enantiomer in the sum of both. Enantioselectivity is commonly alternatively reported in the art (typically as a percentage) as the enantiomeric excess (*e.e*.), calculated therefrom according to the equation [amount of major enantiomer-amount of minor enantiomer]/[amount of major enantiomer+amount of minor enantiomer]. Where the stereoisomers are diastereoisomers, the stereoselectivity is referred to as diastereoselectivity, the fraction (typically reported as a percentage) of one diastereomer in a mixture of two diastereomers, commonly alternatively reported as the diastereomeric excess (*d.e*.). Where a mixture contains more than two diastereomers it is common to report the ratio of diastereomers or "diastereomeric ratio" rather than diastereomeric excess. Thus, enantiomeric excess and diastereomeric excess are types of stereomeric excess. In a more preferred embodiment of the invention, the compound of the formula (III) is formed enriched in one enantiomer and/or enriched in one diastereomer thereof. In other words, the formation of one enantiomer and/or one diastereomer of the compound of the formula (III) is favoured over another (*i.e.* it is formed as a mixture of enantiomers and/or diastereomers thereof whereby one enantiomer and/or one diastereomer thereof is present in greater percentage) or is complete where only one enantiomer or one diastereomer of the compound of the formula (III) is formed.

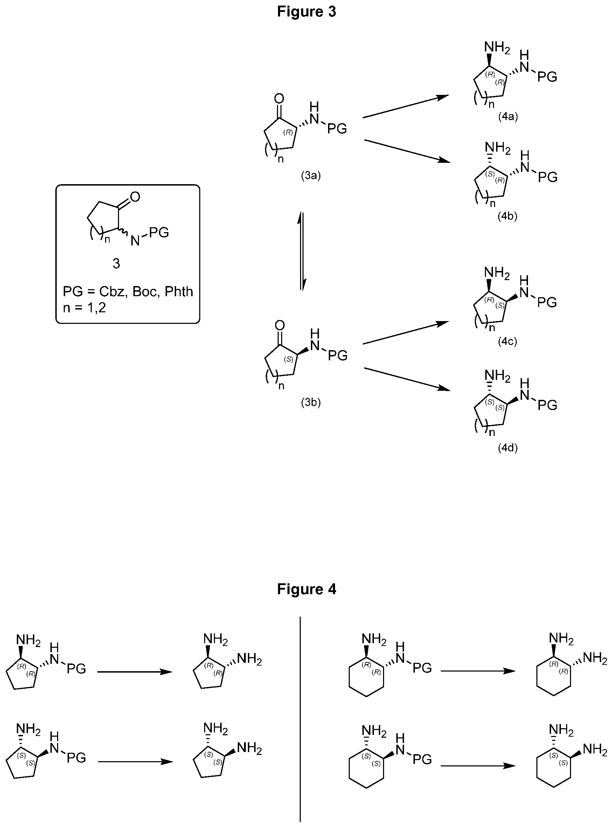

Thus, the term "enantiomerically enriched", as used in the present invention, applies to a mixture of enantiomers of formula (III), wherein one of the enantiomers is present in excess to the other enantiomer or where only one enantiomer of the compound of the formula (III) is formed enantiomerically pure. Therefore, the enantiomerically enriched mixtures have an enantiomeric excess preferably above 80%, more preferably greater than 90%, even more preferably above 95% and furthermore preferably greater than 98%. For example, if an enantiomerically enriched amino alcohol such as enantiomer (2a) (see Figure 2), wherein said alcohol moiety is protected with a benzyl alcohol-protecting group, is produced in excess relative to its enantiomer (2d), said compound (2a) is produced in enantiomeric excess and the mixture is enantiomerically enriched with compound (2a). Likewise, if an enantiomerically enriched diamine such as enantiomer (4a) (see Figure 3) is produced in excess relative to its enantiomer (4d), said diamine (4a) is produced in enantiomeric excess and the mixture is enantiomerically enriched with diamine (4a). An "enantiomerically pure" or "enantiopure" compound for the purposes of the present invention relates to a mixture of two enantiomers with an enantiomeric excess preferably above 95%, more preferably greater than 98%, even more preferably greater than 99%, furthermore preferably higher than 99.5%.

Analogously, the term "diastereomerically enriched", as used in the present invention applied to a mixture of diastereomers is referred to a mixture of diastereomers, wherein one of the diastereomers is present in excess to the other diastereomers or where only one diastereomer of the compound of the formula (III) is formed diastereomerically pure. Therefore, the diastereomerically enriched mixtures have an diastereomeric excess preferably of 1% or greater with respect to one of its diastereomers, more preferably above 20%, even more preferably above 40%, still more preferably greater than 80% and furthermore preferably greater than 95%. For example, if a diastereomerically enriched amino alcohol such as diastereomer (2a) (see Figure 2), wherein said alcohol moiety is protected with a benzyl alcohol-protecting group, is produced in excess relative to the other diastereomers (2b), (2c) and (2d), said compound (2a) is produced in diastereomeric excess and the mixture is diastereomerically enriched with compound (2a). Likewise, if a diastereomerically enriched diamine such as diastereomer (4a) (see Figure 3) is produced in excess relative to the other diastereomers (4b), (4c) and (4d), said diamine (4a) is produced in diastereomeric excess and the mixture is diastereomerically enriched with diamine (4a). A "diastereomerically pure" compound for the purposes of the present invention relates to a mixture of two diastereomers with a diastereomeric excess preferably above 95%, more preferably greater than 98%, even more preferably greater than 99%, furthermore preferably higher than 99.5%.

Dynamic kinetic resolution (DKR) in the present invention refers to a type of kinetic resolution where 100% of a racemic compound can be converted into an enantiopure compound. DKR takes advantage of molecules that can be epimerized at a particular center so that the (R) and (S) enantiomers can interconvert throughout the reaction process. Upon epimerization, a particular catalyst can selectively lower the transition state energy of a single enantiomer, leading to greater yield of one reaction pathway over the other.

As mentioned above, some transaminase polypeptides (preferably selected from ATA-007, ATA-013, ATA-024, ATA-025, ATA-033, ATA-301, ATA-303, ATA-412, ATA-415, P2-A07 or P2-B01, more preferably from P2-A07 or P2-B01) useful in the process of the present invention exhibit high stereoselectivity for the *R*-amine products of compounds (2a) and (2c) (see Figure 2) relative to the corresponding *S*-amine products of compounds (2b) and (2d), respectively, converting a racemic mixture of compound (1) [*i.e.* a compound of formula (II)] 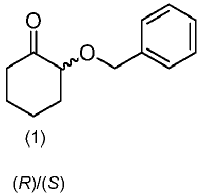 to the *R*-amine products [*i.e.* compounds of formula (III)] in an enantiomeric excess of at least 85% e.e., preferably 90% e.e., more preferably 95% e.e., even more preferably 98% e.e., still more preferably 99% e.e., or more.

Additionally, some of the engineered transaminase polypeptides (preferably selected from ATA-301 or ATA-303) exhibit diastereoselectivity for the trans *R*-amine product and are capable of converting a racemic mixture of compound (1) to the (1*R,*2*R*)-*trans* amine product of compound (2a) in at least a 2:1 diastereomeric ratio relative to the (1*R*,2*S*)*-cis* amine product of compound (2c) under suitable reaction conditions as per Figure 2.

Some transaminase polypeptides (preferably selected from ATA-007, ATA-025, ATA-301, ATA-303, ATA-412 or ATA-415, more preferably ATA-301 or ATA-303) have both high enantioselectivity for *R*-amine products and high diastereoselectivity for the *trans* relative to the *cis* amine products. In a preferred embodiment, the transaminase polypeptides are capable of converting the substrate of compound (1) to compound (2a) in the presence of a substrate concentration of at least about 20 mM, with a percent conversion of at least about 40%, in a reaction time of about 48 h or less, under suitable reaction conditions as specified herein. In a more preferred embodiment the transaminase polypeptides are capable of converting the substrate of compound (1 a) to compound (2a) in the presence of a substrate concentration of at least about 60 mM, with a percent conversion of at least about 60%, in a reaction time of about 36 h or less, under suitable reaction conditions. In an even more preferred embodiment the transaminase polypeptides are capable of converting the substrate of compound (1a) to compound (2a) in the presence of a substrate concentration of at least about 100 mM, with a percent conversion of at least about 90%, in a reaction time of about 24 h or less, under suitable reaction conditions.

Similarly, some of the engineered transaminase polypeptides [preferably selected from ATA-013, ATA-024, ATA-025, ATA-412, ATA-415, ATA-P2-A07 or ATA-P2-B01, more preferably selected from ATA-013, ATA-024, ATA-025, ATA-412, ATA-415 when step (b) is conducted at pH 9.5 to 11.5 and selected from ATA-P2-A07 or ATA-P2-B01 when step (b) is conducted at pH 7.5 to 10] exhibit diastereoselectivity for the *cis R*-amine product of formula IIIa and are capable of converting a racemic mixture of compound (1) to the (1*R*,2*S*)*-cis* amine product of compound (2c) in at least a 2:1 diastereomeric ratio relative to the (1*R*,2*R*)-*trans* amine product of compound (2a) under suitable reaction conditions as specified herein. In a preferred embodiment, the transaminase polypeptides are capable of converting the substrate of compound (1 a) to compound (2a) in the presence of a substrate concentration of at least about 20 mM, with a percent conversion of at least about 40%, in a reaction time of about 48 h or less, under suitable reaction conditions. In a more preferred embodiment the transaminase polypeptides are capable of converting the substrate of compound (1a) to compound (2c) in the presence of a substrate concentration of at least about 60 mM, with a percent conversion of at least about 60%, in a reaction time of about 36 h or less, under suitable reaction conditions. In an even more preferred embodiment the transaminase polypeptides are capable of converting the substrate of compound (1a) to compound (2c) in the presence of a substrate concentration of at least about 100 mM, with a percent conversion of at least about 90%, in a reaction time of about 24 h or less, under suitable reaction conditions.

Similarly, some of the engineered transaminase polypeptides (preferably selected from ATA-113, ATA-251, ATA-254, ATA-256, ATA-260, ATA-P1-A06, ATA-P1-B04 or ATA-P1-G06, more preferably selected from ATA-113, ATA-251, ATA-254, ATA-256, ATA-P1-A06 or ATA-P1-G06) exhibit diastereoselectivity for the *cis S*-amine product of formula IIIa and are capable of converting a racemic mixture of compound (1) to the (1*S*,2*R*)-*cis* amine product of compound (2b) in at least a 2:1 diastereomeric ratio relative to the (1*S,*2*S*)*-trans* amine product of compound (2d) under suitable reaction conditions as specified herein. In a preferred embodiment, the transaminase polypeptides are capable of converting the substrate of compound (1 a) to compound (2b) in the presence of a substrate concentration of at least about 20 mM, with a percent conversion of at least about 40%, in a reaction time of about 48 h or less, under suitable reaction conditions. In a more preferred embodiment the transaminase polypeptides are capable of converting the substrate of compound (1a) to compound (2b) in the presence of a substrate concentration of at least about 60 mM, with a percent conversion of at least about 60%, in a reaction time of about 36 h or less, under suitable reaction conditions. In an even more preferred embodiment the transaminase polypeptides are capable of converting the substrate of compound (1a) to compound (2b) in the presence of a substrate concentration of at least about 100 mM, with a percent conversion of at least about 90%, in a reaction time of about 24 h or less, under suitable reaction conditions.

Similarly, some of the engineered transaminase polypeptides (preferably selected from ATA-217, ATA-234, ATA-238, ATA-P1-F03 or ATA-P1-G05) exhibit diastereoselectivity for the *trans S*-amine product of formula IIIa and are capable of converting a racemic mixture of compound (1) to the (1*S*,2*S*)*-trans* amine product of compound (2d) in at least a 2:1 diastereomeric ratio relative to the (1*S*,2*R)-cis* amine product of compound (2b) under suitable reaction conditions as specified herein. In a preferred embodiment, the transaminase polypeptides are capable of converting the substrate of compound (1a) to compound (2d) in the presence of a substrate concentration of at least about 20 mM, with a percent conversion of at least about 40%, in a reaction time of about 48 h or less, under suitable reaction conditions. In a more preferred embodiment the transaminase polypeptides are capable of converting the substrate of compound (1a) to compound (2d) in the presence of a substrate concentration of at least about 60 mM, with a percent conversion of at least about 60%, in a reaction time of about 36 h or less, under suitable reaction conditions. In an even more preferred embodiment the transaminase polypeptides are capable of converting the substrate of compound (1a) to compound (2d) in the presence of a substrate concentration of at least about 100 mM, with a percent conversion of at least about 90%, in a reaction time of about 24 h or less, under suitable reaction conditions.

Similarly, some of the engineered transaminase polypeptides (preferably selected from ATA-217, ATA-234, ATA-238, ATA-P1-F03 or ATA-P1-G05, more preferably selected from ATA-217, ATA-234 or ATA-238) exhibit diastereoselectivity for the *trans R*-amine product of formula IIIa and are capable of converting a racemic mixture of compound (1) to the (1*R*,2*R)-trans* amine product of compound (2d) in at least a 2:1 diastereomeric ratio relative to the (1*R*,2*S*)*-cis* amine product of compound (2b) under suitable reaction conditions as specified herein. In a preferred embodiment, the transaminase polypeptides are capable of converting the substrate of compound (1a) to compound (2d) in the presence of a substrate concentration of at least about 20 mM, with a percent conversion of at least about 40%, in a reaction time of about 48 h or less, under suitable reaction conditions. In a more preferred embodiment the transaminase polypeptides are capable of converting the substrate of compound (1 a) to compound (2d) in the presence of a substrate concentration of at least about 60 mM, with a percent conversion of at least about 60%, in a reaction time of about 36 h or less, under suitable reaction conditions. In an even more preferred embodiment the transaminase polypeptides are capable of converting the substrate of compound (1 a) to compound (2d) in the presence of a substrate concentration of at least about 100 mM, with a percent conversion of at least about 90%, in a reaction time of about 24 h or less, under suitable reaction conditions.

As mentioned above, compounds (1a) and compound (1 b) are enantiomers that are capable of undergoing an epimerization reaction that provides an equilibrium between them (see figure 2) under certain conditions (*e.g.* preferably pH >9 and temperature >50° C). Because the engineered transaminase polypeptides of the present disclosure exhibit a highly stereoselective preference for the substrate of compound (1a) or compound (1b), this equilibrium between the two enantiomers provides for the ability to carry out a dynamic kinetic resolution of the two enantiomers. Accordingly, in some embodiments of the process the suitable reaction conditions comprise a mixture of an initial amount of the substrate compound (1 a) with its opposite enantiomer of compound (1 b) in the reaction solution in contact with the polypeptide, and the amount of one of the following products: (2a), (2b), (2c) or (2d) formed by the reaction is greater than the starting amount (*i.e.* initial substrate loading) of the corresponding compound (1a) or (1b) from which the compound of formula (IIIa) directly derives via a transamination reaction according to step (b). In some embodiments, where the suitable reaction conditions comprise a racemic mixture of compound (1) in the reaction solution, the yield of one of the following products: (2a), (2b), (2c) or (2d) formed by the reaction relative to the starting amount of the compound (1) is preferably greater than 50%, more preferably greater than 60%, even more preferably greater than 70%, still more preferably greater than 80%, furthermore preferably greater than 90%, even furthermore preferably greater than 95%, or more.

In some embodiments of the process, the suitable reaction conditions comprise a substrate compound (1) [*i.e.* of formula (IIa)] loading of preferably at least about 20 mM, more preferably at least about 40 mM, even more preferably at least about 60 mM, still more preferably at least about 80 mM, furthermore preferably at least about 100 mM, or even greater. In embodiments of the process wherein a racemic mixture of compound (1) is used, the suitable reaction conditions comprise a substrate of compound (1) loading of preferably at least about 20 mM, more preferably at least about 40 mM, even more preferably at least about 60 mM, still more preferably at least about 80 mM, furthermore preferably at least about 100 mM, or even greater. The values for substrate loadings provided herein are based on the molecular weight of compound (1), however it also contemplated that the equivalent molar amounts of various hydrates and salts of compound (1) also can be used in the methods.

The process of transamination is performed employing an amino donor, in some embodiments preferably isopropylamine (IPM). In some embodiments and when the amino donor is IPM, the suitable reaction conditions comprise an IPM concentration of preferably at least about 0.5 M, more preferably at least about 1.0 M, even more preferably at least about 2.5 M, still more preferably at least about 5.0 M, furthermore preferably at least about 7.5 M, even furthermore preferably at least about 10.0 M, or more. In some embodiments, when IPM is used as the amino donor, the process further comprises removal of the carbonyl by-product acetone which is formed from the isopropylamine.

In certain embodiments of the process, the temperature of the suitable reaction conditions can be chosen to maximize the reaction rate at higher temperatures while maintaining the activity of the enzyme for sufficient duration for efficient conversion of the substrate to the product. Higher temperatures increase the rate of epimerization of compound (1 b) to compound (1 a), and thereby allow for a dynamic kinetic resolution process that provides increased product yield of, depending on the employed transaminase, compound (2a), (2b), (2c) or (2c) [*i.e.* compounds of formulae (IIIa)], from a mixture of the substrate compound (1 a) with it opposite enantiomer, compound (1 b) [*i.e.* compounds of formulae (IIa)]. Where higher temperatures are used, polypeptides with increased thermostability can be selected to carry out the process. This allows the engineered polypeptides to be used in methods for converting compound (1a) to compound (2a) at higher temperatures which can result in increased conversion rates and improved substrate solubility characteristics for the reaction, although substrate or product degradation at higher temperatures can contribute to decreased process yields. In some embodiments of the method the suitable reaction conditions comprise a temperature of preferably between about 25 °C and about 75 °C, more preferably between about 35 °C and about 65 °C, even more preferably between about 40 °C and about 60 °C, still more preferably at least about 45 °C, or furthermore preferably at least about 50 °C. In certain embodiments, the temperature during the enzymatic reaction can be maintained at a temperature throughout the course of the reaction. In some embodiments, the temperature during the enzymatic reaction can be adjusted over a temperature profile during the course of the reaction.

The methods for preparing, depending on transaminase involved, compound (2a), (2b), (2c) or (2d) of the present disclosure [*i.e.* compounds of formulae (IIIa)] are generally carried out in a solvent. Suitable solvents include water, aqueous buffer solutions, organic solvents, and/or co-solvent systems, which generally comprise aqueous solvents and organic solvents.

In certain embodiments, the process for preparing compounds (2a), (2b), (2c) or (2d) [*i.e.* compounds of formulae (IIIa)] using the engineered transaminase polypeptides of the present disclosure can be carried out with the pH of the reaction mixture may be maintained at a desired pH or within a desired pH range by the addition of an acid or a base during the course of the reaction. In certain embodiments of the process, the pH of the reaction mixture may be allowed to change, or be changed during the course of the reaction. Thus, it is contemplated that in some embodiments the pH may be controlled by using an aqueous solvent that comprises a buffer. Suitable buffers to maintain desired pH ranges are known in the art and include, for example, phosphate buffer, triethanolamine buffer, and the like. Combinations of buffering and acid or base addition may also be used. In some embodiments of the method the suitable reaction conditions comprise a solution pH of preferably between about pH 8.5 and about pH 11.5, more preferably between about pH 9.0 and about pH 11.5, even more preferably between about pH 9.5 and about pH 11.0, still more preferably at least about pH 10.0, or furthermore preferably at least about pH 10.5.

During the course of the transamination reactions, the pH of the reaction mixture may change. The pH of the reaction mixture may be maintained at a desired pH or within a desired pH range by the addition of an acid or a base during the course of the reaction. Alternatively, the pH may be controlled by using an aqueous solvent that comprises a buffer. Suitable buffers to maintain desired pH ranges are known in the art and include, for example, phosphate buffer, triethanolamine buffer, and the like. Combinations of buffering and acid or base addition may also be used. In some embodiments, the buffer is TEA (*e.g*. about 0.025 M to about 0.25 M TEA). In some embodiments of the process the suitable reaction conditions comprise a buffer solution of about 0.05 M borate to about 0.25 M borate, or about 0.1 M borate. In some embodiments, the reaction conditions comprise water as a suitable solvent with no buffer present.

In some embodiments, the process for preparing compound (2a), (2b), (2c) or (2d) [*i.e.* compounds of formulae (IIIa)] using an engineered transaminase polypeptide described are generally carried out in an aqueous co-solvent system comprising an organic solvent [*e.g*. ethanol, isopropanol (IPA), dimethyl sulfoxide (DMSO), ethyl acetate, butyl acetate, 1-octanol, heptane, octane, methyl t-butyl ether (MTBE), toluene, and the like] and/or ionic liquids (*e.g*. 1-ethyl 4-methylimidazolium tetrafluoroborate, 1-butyl-3-methylimidazolium tetrafluoroborate, 1-butyl-3-methylimidazolium hexafluorophosphate, and the like). The organic solvent component of an aqueous co-solvent system may be miscible with the aqueous component, providing a single liquid phase, or may be partly miscible or immiscible with the aqueous component, providing two liquid phases. Exemplary aqueous co-solvent systems comprise water and one or more organic solvent. In general, an organic solvent component of an aqueous co-solvent system is selected such that it does not completely inactivate the transaminase enzyme. Appropriate co-solvent systems can be readily identified by measuring the enzymatic activity of the specified engineered transaminase enzyme with a defined substrate of interest in the candidate solvent system, utilizing an enzyme activity assay, such as those described herein. In some embodiments of the process, the suitable reaction conditions comprise an aqueous co-solvent comprising DMSO at a concentration of preferably at least about 5% (v/v), more preferably at least about 10% (v/v), even more preferably at least about 20% (v/v), still more preferably at least about 30% (v/v), or furthermore preferably at least about 40% (v/v).

The suitable reaction conditions used in the process can comprise a combination of reaction parameters that provide for the biocatalytic conversion of compound (1a) [*i.e.* of formulae (IIa)] to, depending of the involved transaminase, compound (2a), (2b), (2c) or (2d) [*i.e.* compounds of formulae (IIIa)] in a higher diastereomeric ratio and in a higher percentage conversion. Accordingly, in a preferred embodiment of the process, the combination of reaction parameters comprises: (a) substrate loading of about 20 - 100 mM compound (1 a); (b) polypeptide concentration of about 1.0 - 20 g/L; (e) IPM concentration of about 0.1 - 10 M; (d) PLP cofactor at a concentration of about 0.1 - 1.0 mM; (e) about pH 8.5 - 11.0; and (f) temperature of about 30 - 60 °C. In a more preferred embodiment, the combination of reaction parameters comprises: (a) at least 20 mM compound (1a); (b) at least 1 g/L polypeptide; (c) at least 1 M isopropylamine; (d) at least 1 mM PLP; (e) about 0.15 - 0.25 M borate; (f) at least 20% (v/v) DMSO; (g) pH 10 - 11; and (h) a temperature of 40 to 50 °C.

In one embodiment of the method, the suitable reaction conditions comprise a substrate loading of compound (1 a) of preferably at least about 20 mMand wherein the method results in at least about 90% conversion of compound (1 a) to compound (2a) in about 48 h or less. In another more preferred embodiment, the reaction conditions comprise a substrate loading of compound (1 a) of at least about 60 mM, and wherein the method results in at least about 95% or greater conversion of compound (1a) to compound (2a) in about 36 h or less. In an even more preferred embodiment, the reaction conditions comprise a substrate loading of compound (1 a) of at least 80 mM, and wherein the method results in at least about 97% or greater conversion of compound (1a) to compound (2a) in about 24 h or less.

In a preferred embodiment of the process, the suitable reaction conditions comprise a substrate loading of compound (1a)as described herein, and the method results in the conversion of the racemic mixture of compound (1) [*i.e*. of formulae (IIa)] to the corresponding product (2a) (2a), (2b), (2c) or (2d) [*i.e.* compounds of formulae (IIIa)], depending on the transaminase involved, in a diastereomeric ratio of at least about 2:1, more preferably at least about 3:1, even more preferably at least about 4:1, still more preferably at least about 8:1, furthermore preferably at least about 20:1, even furthermore preferably at least about 30:1. Further, where the suitable reaction conditions suitable that allow for the epimerization of compound (1 b) to compound (1 a), the method can provide a dynamic kinetic resolution and the yield of the corresponding product [(2a), (2b), (2c) or (2d), depending on the involved transaminase] formed by the reaction relative to the starting amount of the compound (1) is preferably greater than 50%, more preferably greater than 60%, even more preferably greater than 70%, still more preferably greater than 80%, furthermore preferably greater than 90%, even furthermore preferably greater than 95%.

In carrying out the transamination reactions described in the process of the instant invention, the engineered transaminase polypeptide may be added to the reaction mixture in the form of a purified enzyme, whole cells transformed with gene(s) encoding the enzyme, and/or as cell extracts and/or lysates of such cells. Whole cells transformed with gene(s) encoding the engineered transaminase enzyme or cell extracts, lysates thereof, and isolated enzymes may be employed in a variety of different forms, including solid (*e.g.* lyophilized, spray-dried, and the like) or semisolid (*e.g.* a crude paste). The cell extracts or cell lysates may be partially purified by precipitation (ammonium sulfate, polyethyleneimine, heat treatment or the like, followed by a desalting procedure prior to lyophilization (*e.g*. ultrafiltration, dialysis, and the like). Any of the cell preparations may be stabilized by crosslinking using known crosslinking agents, such as, for example, glutaraldehyde or immobilization to a solid phase (*e.g*. Eupergit C, and the like). In some embodiments where the engineered polypeptide can be expressed in the form of a secreted polypeptide and the culture medium containing the secreted polypeptides can be used in the method of converting compound (1a) to compound (2a), (2b), (2c) or (2d).

In some embodiments, solid reactants (*e.g.* enzyme, salts, etc.) may be provided to the reaction in a variety of different forms, including powder (*e.g*. lyophilized, spray dried, and the like), solution, emulsion, suspension, and the like. The reactants can be readily lyophilized or spray dried using methods and equipment that are known to those having ordinary skill in the art. For example, the protein solution can be frozen at -80 °C. in small aliquots, then added to a pre-chilled lyophilization chamber, followed by the application of a vacuum.

The quantities of reactants used in the transamination reaction will generally vary depending on the quantities of product desired, and concomitantly the amount of transaminase substrate employed. Those having ordinary skill in the art will readily understand how to vary these quantities to tailor them to the scale of production. In general, the transaminase substrates are kept at levels that achieve essentially complete or near complete conversion of the substrates into products. Generally, the transamination reaction is generally allowed to proceed until essentially complete, or near complete, transformation of substrate is obtained. Transformation of substrate to product can be monitored using known methods by detecting substrate and/or product. Suitable methods include gas chromatography, HPLC, and the like.

In some embodiments, the process can further comprise a step of removal of the carbonyl by-product formed from the amino group donor when the amino group is transferred to the substrate of compound (1). Such removal in situ can reduce the rate of the reverse reaction such that the forward reaction dominates and more substrate is then converted to product. Removal of the carbonyl by-product can be carried out in a number of ways. Where the amino group donor is an amino acid, such as alanine, the carbonyl by-product, a keto acid, can be removed by reaction with a peroxide such as, among others, hydrogen peroxide; peroxyacids (peracids) such as peracetic acid (CH₃CO₃H), trifluoroperacetic acid and metachloroperoxybenzoic acid; organic peroxides such as *t*-butyl peroxide (CH₃)₃COOH), or other selective oxidants such as tetrapropylammonium perruthenate, MnO₂, KMnO₄, ruthenium tetroxide and related compounds. Alternatively, pyruvate removal can be achieved via its reduction to lactate by employing lactate dehydrogenase to shift equilibrium to the product and pyruvate removal can be achieved via its decarboxylation to carbon dioxide acetaldehyde by employing pyruvate decarboxylase.

In some embodiments of the process, where the choice of the amino donor results in a carbonyl by-product that has a vapor pressure higher than water (*e.g*. a low boiling co-product such as a volatile organic carbonyl compound), the carbonyl by-product can be removed by sparging the reaction solution with a non-reactive gas or by applying a vacuum to lower the reaction pressure and removing the carbonyl by-product present in the gas phase. A non-reactive gas is any gas that does not react with the reaction components. Various non-reactive gases include nitrogen and noble gases (*e.g*. inert gases). In some embodiments, the non-reactive gas is nitrogen gas.

In some preferred embodiments, the amino donor used in the process is isopropylamine (IPM), which forms the carbonyl by-product acetone upon transfer of the amino group to the amino group acceptor. The acetone can be removed by sparging with nitrogen gas or applying a vacuum to the reaction solution and removing the acetone from the gas phase by an acetone trap, such as a condenser or other cold trap. Alternatively, the acetone can be removed by reduction to isopropanol using a ketoreductase.

In some embodiments of the process where the carbonyl by-product is removed, the corresponding amino group donor can be added during the transamination reaction to replenish the amino group donor and/or maintain the pH of the reaction. Replenishing the amino group donor also shifts the equilibrium towards product formation, thereby increasing the conversion of substrate to product. Thus, in some embodiments wherein the amino group donor is IPM and the acetone product is removed *in situ,* the method can further comprise a step of adding IPM to the reaction solution to replenish the amino group donor lost during the acetone removal and to maintain the pH of the reaction (*e.g*. at about 8.0 to 9.0).

Alternatively, in embodiments where an amino acid is used as amino group donor, the keto acid carbonyl by-product can be recycled back to the amino acid by reaction with ammonia and NADH using an appropriate amino acid dehydrogenase enzyme, thereby replenishing the amino group donor.

In some embodiments, the process comprises the step of an enzymatic conversion of compound (1) [*i.e*. compound of formulae (IIa)]to, depending on the involved transaminase, compound (2a), (2b), (2c) or (2d) [*i.e*. compounds of formulae (IIIa)] using an engineered transaminase polypeptide can further comprise chemical steps of product work-up, extraction, isolation, purification, and/or crystallization, each of which can be carried out under a range of conditions.

In some embodiments, the present disclosure also contemplates that the process comprising the step of an enzymatic conversion of compound (1) to compound (2a) using an engineered transaminase polypeptide can further comprise one or more further chemical steps for converting compound (2a) to (1*R*,2*R*)-2-aminocyclohexanol, or its salts, hydrates, or solvates.

In some embodiments, the present disclosure also contemplates that the process comprising the step of an enzymatic conversion of compound (1) to compound (2b) using an engineered transaminase polypeptide can further comprise one or more further chemical steps for converting compound (2b) to (1*R*,2*S*)-2-aminocyclohexanol, or its salts, hydrates, or solvates.

In some embodiments, the present disclosure also contemplates that the process comprising the step of an enzymatic conversion of compound (1) to compound (2c) using an engineered transaminase polypeptide can further comprise one or more further chemical steps for converting compound (2c) to (1*S*,2*R*)-2-aminocyclohexanol, or its salts, hydrates, or solvates.

In some embodiments, the present disclosure also contemplates that the process comprising the step of an enzymatic conversion of compound (1) to compound (2d) using an engineered transaminase polypeptide can further comprise one or more further chemical steps for converting compound (2d) to (1 S,2S)-2-aminocyclohexanol, or its salts, hydrates, or solvates.

In some preferred embodiments, the conversion of compound (1) [*i.e.* compound of formulae (IIa)] to, depending on the involved transaminase, compound (2a), (2b), (2c) or (2d) [*i.e.* compounds of formulae (IIIa)] can be carried out wherein the method comprises contacting an analog of compound (1) (Figure 2) with an engineered transaminase polypeptide in the presence of an amino donor under suitable reaction conditions, thereby resulting in the preparation of the chiral amine of the corresponding analog of product compounds (2a), (2b), (2c) or (2d) in diastereomeric excess. Suitable reactions conditions for the conversion of analogs of compound (1) to the chiral amine of the corresponding analogs of compound (2a), (2b), (2c) or (2d) can be the same as used for compound (1) or determined by the ordinary artisan based on the known properties of the analog compounds and routine experimentation.

In furthermore preferred special embodiment, the analog of compound (1) which is submitted to reaction with an engineered transaminase polypeptide in the presence of an amino donor under suitable reaction conditions is a compound of formula (3) which bears a protected amine in a contiguous position (*i.e.* α-position) to the ketone group (Figure 3). Depending on the involved transaminase and by means of a DKR process, (3) [*i.e*. compound of formula (Ila)] was transformed into compound (4a), (4b), (4c) or (4d) [*i.e*. compounds of formulae (IIIa)] in high enantio- and diastereomeric excess. Suitable reactions conditions for the conversion of (3) into any of the compounds (4a), (4b), (4c) or (4d) can be the same as used for compound (1) or determined by the ordinary artisan based on the known properties of the analog compounds and routine experimentation.

In some embodiments, the present disclosure also contemplates the cleavage of the protecting groups (PG) in the molecules (4a), and (4d) to obtain enantiomerically pure *trans* (*R*,*R*)-cyclopentane-1,2-diamine or *trans* (*S,S*)-cyclopentane-1,2-diamine and enantiomerically pure *trans* (*R*,*R*)-cyclohexane 1,2-diamine or *trans* (*S*,*S*)-cyclohexane 1,2-diamine or salts thereof, or to obtain a enantiomerically enriched enantiomer of *trans*-cyclopentane-1,2-diamine or *trans-*cyclohexane-1,2-diamine or salts thereof (Figure 4).

It is a particular strength of the present invention that in one more particularly preferred special embodiment the oxidation of the hydrocarbon comprising a hydroxyl group [*i.e.* the compound of formula (III)] in step a) works by using exclusively 1 mol% of organocatalyst (AZADO) and only one equivalent of sodium hypochlorite to recycle the catalyst addressing the initial concern of developing a green, economical and efficient oxidation system compared to existing methods.

More interestingly, the oxidation system AZADO/NaOCI employed in step a) has a null impact on the activity of the biological agent having transaminase activity when step b) was sequentially coupled.

Thus, one particularly preferred special embodiment of the present invention relates to a method for producing a compound of formula (III) 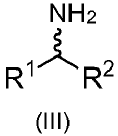 or a salt thereof, wherein said process comprises the following steps:
(a) forming a mixture comprising a compound of formula (II) 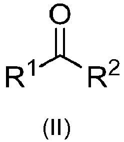 by adding an organocatalyst selected from 2,2,6,6-tetramethylpiperidin-1-oxyl, 4-hydroxy-2,2,6,6-tetramethylpiperidin-1-oxyl, 4-acetamido-2,2,6,6-tetramethylpiperidine-1-oxyl, 9-azabicyclo[3.3.1]nonane-*N*-oxyl, 2,2'-azino-bis(3-ethylbenzothiazoline-6-sulphonic acid), 2-azaadamantane-*N*-oxyl or 1-methyl-2-azaadamantane-*N*-oxyl to a compound of formula (I) 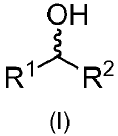 in an aqueous medium comprising NaOCl and an organic co-solvent; and
(b) adding:
   - an aqueous potassium phosphate buffer;
   - an omega-transaminase;
   - an amine donor compound selected from isopropylamine, benzyl amine, (*R*)-1-phenylethylamine, (*S*)-1-phenylethylamine, (*R*)(*S*)-1-phenylethylamine, L-alanine, D-alanine, D,L-alanine or 2-aminoethanol; and
   - a cofactor selected from pyridoxal-5'-phosphate (also known as 1-(4'-formyl-3'-hydroxy-2'-methyl-5'-pyridyl)methoxyphosphonic acid, pyridoxal-phosphate, PLP, PYP, P5P or CAS number [54-47-7]), pyridoxine (PN), pyridoxal (PL), pyridoxamine (PM), pyridoxine phosphate (PNP) or pyridoxamine phosphate (PMP),
   to the mixture obtained in step (a), wherein said transamination catalyst is added after adding said aqueous potassium phosphate buffer, and said aqueous potassium phosphate buffer added in step (b) dilutes the mixture obtained in step (a) by at least 5 times (*i.e.* five-fold),
   wherein:
   (i) R¹ is an R³ substituent and R² is an R³ substituent, wherein each R³ substituent is independently selected from:
      - an R^{3a} substituent;
      - an R^{3b} substituent; or
      - an R^{3a} substituent which is substituted with 1 to 4 substituents each independently selected from an R^{3b} substituent or an R^{3a} substituent that is optionally substituted with
         - an R^{3a} substituent,
         - an R^{3b} substituent, or
         - an R^{3a} substituent that is optionally substituted with Z or an R^{3b} substituent,
      and wherein
      - each R^{3a} substituent is independently selected from a C₁-C₁₂ alkyl or a carbocyclyl moiety wherein said carbocyclyl moiety is a monocyclic or bicyclic C₅-C₉ aryl comprising one aromatic ring;
      - each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN, or -Si(R^{3a})₃,
      - R⁴ is H or R⁶;
      - R⁵ is H or an R^{3a} substituent that is optionally substituted with another R^{3a} substituent;
      - R⁶ is an R^{3a} substituent or -(C=O)-OR⁵; and
      - Z is halogen.
      or
   (ii) R¹ and R², when taken with the carbon atom to which they are both attached, complete a non-aromatic 5-membered to 8-membered ring of a carbocycle or a heterocycle comprising at least one N, S or O atom, wherein said carbocycle or heterocycle comprises one ring or two fused 5-membered to 8-membered rings, and wherein said carbocycle or heterocycle is unsubstituted or substituted with 1 or 2 R³ substituents, wherein each R³ substituent is independently selected from:
      - an R^{3a} substituent;
      - an R^{3b} substituent;
      - an R^{3a} substituent which is substituted with 1 to 4 substituents each independently selected from =O, an R^{3b} substituent or an R^{3a} substituent that is optionally substituted with an R^{3b} substituent; or
      - =O;
      and wherein:
      - each R^{3a} substituent is independently selected from a C₁-C₁₂ alkyl, a carbocyclyl or a heterocyclyl moiety comprising at least one N atom, wherein said carbocyclyl or a heterocyclyl moiety comprises one 5-membered to 8-membered ring or two fused 5-membered to 8-membered rings;
      - each R^{3b} substituent is independently selected from -OR⁵, -(C=O)-R⁵, -(C=O)-OR⁵, -(C=O)-NR⁴R⁶, -O(C=O)-OR⁵, -NR⁴-(C=O)-OR⁵, -O(C=O)-NR⁴R⁶, -NR⁴R⁶, -CN or -Si(R^{3a})₃;
      - R⁴ is H or R⁶;
      - R⁵ is H or an R^{3a} substituent that is optionally substituted with another R^{3a} substituent; and
      - R⁶ is an R^{3a} substituent or -(C=O)-OR⁵.

### Examples

The invention is illustrated in the examples, as well as in the figures and generic schemes. The substituents and integers used in the follow schemes are as defined in the embodiments of the instant invention, unless otherwise indicated. This section is set forth to aid in an understanding of the invention but should not be construed to limit in any way the invention as set forth in the claims.

### Example 1: Oxidation of acyclic and cyclic compounds comprising a hydroxyl group by 2-azaadamantane-N-oxyl (AZADO) and sodium hypochlorite.

A range of acyclic and cyclic compounds comprising a hydroxyl group (Figure 5 and Figure 6) in addition to 1-phenylpropan-1-ol was subjected to organocatalyzed oxidation as follows. To a solution of racemic alcohol (150 µmol) and AZADO (1.5 µmol) in PhCF₃ (150 µL), a 0.40 M solution of NaOCl (pH = 8.9; 1.0 to 1.4 molar equiv.) was added. The mixture was vigorously stirred (magnetic stirring) until disappearance of the starting material (1 h, TLC control using hexane-ethyl acetate 3:1 as eluent). Then, reaction mixture was extracted with ethyl acetate (3 × 600 µL). The organic layers were combined, dried over Na₂SO₄ and evaporated under vacuum. The product propiophenone was isolated pure with a yield of 95%. The results for the oxidation of said range of compounds are presented in Table 1.

**Table 1. Quantitative oxidation of alcohols mediated by aqueous NaOCl and 1% AZADO in 1 h.^{a,b,c}**

| **Alcohol** | **NaOCl (equiv)** | **Ketone** |
|---|---|---|
| 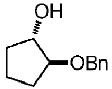 | 1.2 | 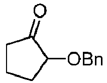 |
| 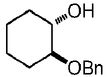 | 1.2 | 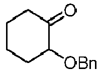 |
| 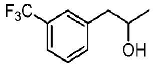 | 1.2 | 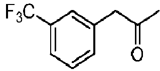 |
| 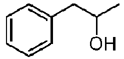 | 1.4 | 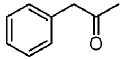 |
| 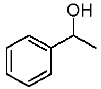 | 1.2 | 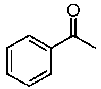 |
| 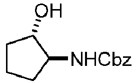 | 1.2 | 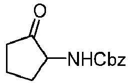 |
| 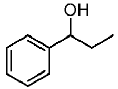 | 1.2 | 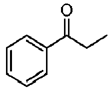 |
| 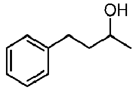 | 1.2 | 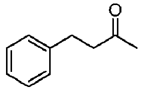 |
| 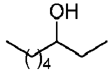 | 1.4 | 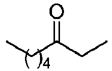 |
| 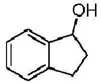 | 1.0 | 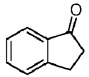 |
| 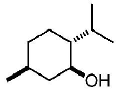 | 1.4 | 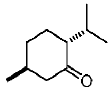 |
| 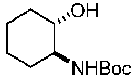 | 1.2 | 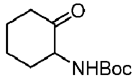 |

| | | |
|---|---|---|
| *^{a}* Reaction conditions: alcohol (150 µmol, 220 to 290 mM), AZADO (1.5 µmol), PhCF₃ (150 µL, 22 to 30% v/v), in a 0.40 M solution of NaOCl (pH = 8.9; 1.0 to 1.4 equiv.) vigorously stirred at a temperature between 5 °C and room temperature (22 °C) for 1 h. *^{b}* Isolated yields >90%. *^{c}* Corresponding to step (a) of the present invention. | | |

### Example 2: Transamination of acyclic prochiral compounds comprising a ketone group.

In a 1.5 mL Eppendorf tube, a range of acyclic compounds (prochiral compounds) comprising a ketone group in addition to propiophenone (20 mM), transaminase (2 mg), and DMSO (50 µL) were added in phosphate buffer (previously degassed with Ar) 100 mM pH 8.5 (500 µL, 1 mM PLP, 1 M IPM). The reaction was shaken at 250 rpm and 30 °C for 24 h and stopped by the addition of 10 N NaOH (100 µL). Then the mixture was extracted with ethyl acetate (2 × 500 µL), the organic layers separated by centrifugation (90 s, 13000 rpm), combined and finally dried over Na₂SO₄. Conversion was determined directly by HPLC in reverse phase and the enantiomeric excess by chiral HPLC after derivatization with di-*tert*-butyl dicarbonate, the results for which are presented in Tables 2 to 4.

**Table 2. Enzymatic transamination of [3-(trifluoromethyl)phenyl]acetone using a given ω-transaminase (ω-TA) at pH 7.5.^{a}**

| | | |
|---|---|---|
| 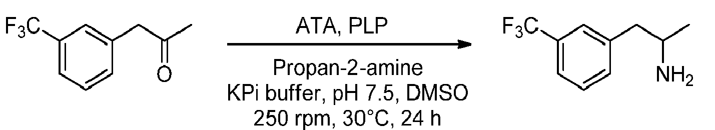 | | |

| **ω-TA** | ***c* (%)***^{b}* | ***ee* (%)***^{c}* |
|---|---|---|
| ATA-007 | 24 | **-** |
| ATA-013 | 99 | 83 *(R)* |
| ATA-024 | 74 | 75 *(R)* |
| ATA-025 | 72 | 63 (*R*) |
| ATA-033 | 72 | 66 (*R*) |
| ATA-113 | 87 | 83 (*S*) |
| ATA-117 | 15 | - |
| ATA-200 | 14 | - |
| ATA-217 | 73 | 73 (*S*) |
| ATA-234 | 84 | 35 (*S*) |
| ATA-237 | >99 | >99 (*S*) |
| ATA-238 | 66 | 80 (*S*) |
| ATA-251 | 99 | 94 (*S*) |
| ATA-254 | 90 | 94 (*S*) |
| ArS | >99 | 92 (*S*) |
| Cv | 18 | - |
| ATA-256 | 99 | 94 (*S*) |
| ATA-260 | 99 | 93 (*S*) |
| ATA-301 | 73 | >99 (*R*) |
| ATA-303 | 77 | 91 (*R*) |
| ATA-412 | 81 | >99 (*R*) |
| ATA-415 | 68 | 90 (R) |
| ATA-P1-A06 | 28 | - |
| ATA-P1-B04 | 58 | 89 (*S*) |
| ATA-P1-F03 | 22 | 71 (*S*) |
| ATA-P1-G05 | 67 | >99 (*S*) |
| ATA-P1-G06 | 39 | - |
| ATA-P2-A01 | 27 | - |
| ATA-P2-A07 | 52 | >99 (*R*) |
| ATA-P2-B01 | >99 | >99 (*R*) |
| ArRmut11 | >99 | 74 (*R*) |
| ArR | 48 | 46 (*R*) |

| | | |
|---|---|---|
| *^{a}* Step (b) of the present invention, wherein [3-(trifluoromethyl)phenyl]acetone is obtained according to step (a) of the present invention. *^{b}* Determined by HPLC analysis *^{c}* Determined by chiral-HPLC analysis. The absolute configuration of the amine is indicated between brackets. | | |

**Table 3. Enzymatic transamination of 1-phenylpropan-2-one using a given ω-transaminase (ω-TA) at pH 7.5.^{a}**

| | | |
|---|---|---|
| 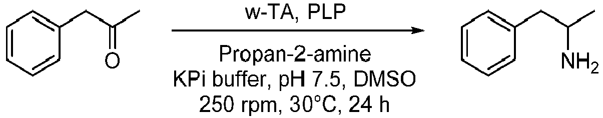 | | |

| **ω-TA** | ***c* (%)*^{b}*** | ***ee* (%)*^{c}*** |
|---|---|---|
| ATA-113 | 96 | 80 (*S*) |
| ATA-237 | 95 | 96 (*S*) |
| ATA-251 | 95 | >99 *(S)* |
| ATA-256 | 57 | >99 *(S)* |
| ArS | 64 | >99 *(S)* |
| Cv | 91 | >99 *(S)* |
| ATA-412 | 83 | >99 (*R*) |
| ATA-P1-F03 | 70 | >99 (*S*) |
| ATA-P1-G06 | 95 | >99 (*S*) |
| | | |
| ArRmut11 | >99 | >99 (*R*) |
| ArR | 91 | >99 (*R*) |

| | | |
|---|---|---|
| *^{a}* Step (b) of the present invention, wherein 1-phenylpropan-2-one is obtained according to step (a) of the present invention. *^{b}* Determined by HPLC analysis *^{c}* Determined by chiral-HPLC analysis. The absolute configuration of the amine is indicated between brackets. | | |

**Table 4. Enzymatic transamination of 4-phenylbutan-2-one using a given ω-transaminase (ω-TA) at pH 7.5.^{a}**

| | | |
|---|---|---|
| 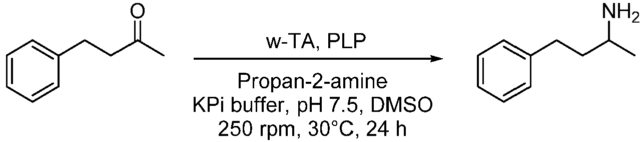 | | |

| **ω-TA** | ***c* (%)*^{b}*** | ***ee* (%)*^{c}*** |
|---|---|---|
| ATA-007 | 28 | <5 |
| ATA-013 | 94 | 40 (*R*) |
| ATA-024 | >99 | 12 (*R*) |
| ATA-025 | >99 | 20 (*R*) |
| ATA-033 | >99 | 22 (*R*) |
| ATA-113 | >99 | 80 (*S*) |
| ATA-117 | 82 | >99 (*R*) |
| ATA-200 | 51 | >99 (*S*) |
| ATA-217 | 87 | 64 (*S*) |
| ATA-234 | 64 | >99 (*S*) |
| ATA-237 | >99 | >99 (*S*) |
| ATA-238 | 58 | >99 (*S*) |
| ATA-251 | >99 | >99 (*S*) |
| ATA-254 | >99 | >99 (*S*) |
| ArS | >99 | >99 (*S*) |
| Cv | >99 | >99 (*S*) |
| ATA-256 | 89 | >99 (*S*) |
| ATA-260 | 87 | >99 (*S*) |
| ATA-301 | 56 | 68 (*R*) |
| ATA-303 | 78 | 54 (R) |
| ATA-412 | 56 | >99 (*R*) |
| ATA-415 | >99 | 28 (*R*) |
| ATA-P1-A06 | >99 | >99 (*S*) |
| ATA-P1-B04 | >99 | >99 (*S*) |
| ATA-P1-F03 | 91 | >99 (*S*) |
| ATA-P1-G05 | >99 | >99 (*S*) |
| ATA-P1-G06 | >99 | >99 (*S*) |
| ATA-P2-A01 | 60 | >99 (*R*) |
| ATA-P2-A07 | >99 | >99 (*R*) |
| ATA-P2-B01 | >99 | >99 (*R*) |
| ArRmut11 | >99 | 36 (*R*) |
| ArR | >99 | >99 (*R*) |

| | | |
|---|---|---|
| *^{a}* Step (b) of the present invention, wherein 4-phenylbutan-2-one is obtained according to step (a) of the present invention. *^{b}* Determined by HPLC analysis *^{c}* Determined by chiral-HPLC analysis. The absolute configuration of the amine is indicated between brackets. | | |

### Example 3: Transamination of racemic cyclic compounds involving a DKR and comprising a ketone group.

In a 1.5 mL Eppendorf tube, a range of racemic cyclic compounds (containing one stereocenter) comprising a ketone group in addition to 2-(benzyloxy)cyclohexanone (20 mM), transaminase (2 mg), and DMSO (50 µL) were added to a mixture of 500 µL of 100 mM phosphate buffer at pH 8.5, 1 mM PLP, and 1 M IPM). The reaction was shaken at 250 rpm and 45 °C for 24 h. Then the mixture was extracted with ethyl acetate (2 × 500 µL), the organic layers separated by centrifugation (90 sec, 13000 rpm), combined and finally dried over Na₂SO₄. Conversion and diastereomeric ratio were determined directly by HPLC in reverse phase and the enantiomeric excess by chiral HPLC after derivatization with di-*tert*-butyl decarbonate. In addition to the reaction at pH 8.5, the results for which are presented in Table 5, the same reaction was carried out at pH 10 and pH 11.5, the results for which are presented in Tables 6 to 8, respectively.

**Table 5. Enzymatic amination of 2-(benzyloxy)cyclohexanone using a given ω-transaminase (ω-TA) at pH 8.5.^{a}**

| | | | | | |
|---|---|---|---|---|---|
| 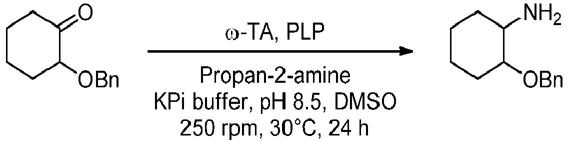 | | | | | |

| **ω-TA** | ***c* (%)*^{b}*** | ***dr* (%)***^{b}* | | ***ee* (%)***^{b}* | |
|---|---|---|---|---|---|
| | | ***cis*** | ***trans*** | ***cis*** | ***trans*** |
| ATA-007 | >99 | 48 | 52 | >99 (1*R*,2*S*) | >99 (1*R,*2*R*) |
| ATA-013 | >99 | 49 | 51 | >99 (1*R*,2*S*) | >99 (1*R,*2*R*) |
| ATA-024 | 80 | 49 | 51 | >99 (1*R*,2*S*) | >99 (1*R,*2*R*) |
| ATA-025 | >99 | 48 | 52 | >99 (1*R*,2*S*) | >99 (1*R,*2*R*) |
| ATA-033 | 62 | 50 | 50 | >99 (1*R*,2*S*) | >99 (1*R,*2*R*) |
| ATA-113 | >99 | 65 | 35 | >99 (1*S*,2*R*) | >99 (1*S,*2*S*) |
| ATA-217 | >99 | 48 | 52 | >99 (1*S*,2*R*) | >99 (1*S,*2*S*) |
| ATA-234 | >99 | 47 | 53 | >99 (1*S*,2*R*) | >99 (1*S*,2*S*) |
| ATA-237 | >99 | 51 | 49 | >99 (1*S*,2*R*) | >99 (1*S*,2*S*) |
| ATA-238 | >99 | 49 | 51 | >99 (1*S*,2*R*) | >99 (1*S,*2*S*) |
| ATA-251 | >99 | 63 | 37 | >99 (1*S*,2*R*) | >99 (1*S,2S*) |
| ATA-254 | >99 | 74 | 26 | >99 (1*S*,2*R*) | >99 (1*S,2S*) |
| ATA-256 | >99 | 76 | 24 | >99 (1*S*,2*R*) | >99 (1*S,2S*) |
| ATA-260 | >99 | 51 | 49 | >99 (1*S*,2*R*) | >99 (1*S,2S*) |
| ATA-301 | >99 | 45 | 55 | >99 (1*R*,2*S*) | >99 (1*R,*2*R*) |
| ATA-303 | >99 | 48 | 52 | >99 (1*R*,2*S*) | >99 (1*R,*2*R*) |
| ATA-412 | >99 | 49 | 51 | >99 (1*R*,2*S*) | >99 (1*R,*2*R*) |
| ATA-415 | >99 | 46 | 54 | >99 (1*R*,2*S*) | >99 (1*R,*2*R*) |
| ATA-P1-A06 | 59 | 78 | 22 | >99 (1*S*,2*R*) | >99 (1*S,2S*) |
| ATA-P1-B04 | >99 | 53 | 47 | >99 (1*S*,2*R*) | >99 (1*S*.2*S*) |
| ATA-P1-F03 | >99 | 49 | 51 | >99 (1*S*,2*R*) | >99 (1*S*.2*S*) |
| ATA-P1-G05 | >99 | 49 | 51 | >99 (1*S*,2*R*) | >99 (1*S,2S*) |
| ATA-P1-G06 | 82 | 65 | 35 | >99 (1*S*,2*R*) | >99 (1*S,2S*) |
| ATA-P2-A07 | 77 | 77 | 23 | >99 (1*R*,2*S*) | >99 (1*R,*2*R*) |
| ATA-P2-B01 | 45 | 92 | 8 | >99 (1*R*,2*S*) | |

| | | | | | |
|---|---|---|---|---|---|
| *^{a}* Step (b) of the present invention, wherein 2-(benzyloxy)cyclohexanone is obtained according to step (a) of the present invention. *^{b}*Determined by HPLC analysis. | | | | | |

**Table 6. Enzymatic transamination of (±)-2-benzyloxycyclohexanone using a given ω-transaminase (ω-TA) at pH 10.^{a}**

| | | | |
|---|---|---|---|
| 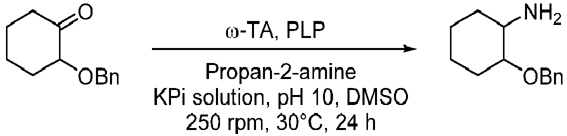 | | | |

| **ω-TA** | ***c*(%)*^{b}*** | ***dr*(%)***^{b}* | |
|---|---|---|---|
| | | ***cis**^{c}* | ***trans**^{c}* |
| ATA-013 | >99 | 51 | 49 |
| ATA-024 | 80 | 49 | 51 |
| ATA-025 | 84 | 50 | 50 |
| ATA-033 | 79 | 49 | 51 |
| ATA-217 | >99 | 46 | 54 |
| ATA-234 | >99 | 47 | 53 |
| ATA-237 | >99 | 51 | 49 |
| ATA-238 | >99 | 48 | 51 |
| ATA-251 | 98 | 79 | 21 |
| ATA-254 | 89 | 85 | 15 |
| ATA-256 | 62 | 86 | 14 |
| ATA-260 | >99 | 74 | 26 |
| ATA-301 | >99 | 33 | 67 |
| ATA-303 | >99 | 32 | 68 |
| ATA-412 | >99 | 50 | 50 |
| ATA-415 | >99 | 43 | 57 |
| ATA-P1-B04 | 40 | 63 | 37 |
| ATA-P1-F03 | >99 | 48 | 52 |
| ATA-P1-G05 | >99 | 52 | 48 |
| ATA-P2-B01 | 32 | 92 | 8 |
| ATA-P1-A06 | 77 | 82 | 18 |
| ATA-P1-G06 | 66 | 79 | 21 |
| ATA-P2-A07 | 90 | 87 | 13 |

| | | | |
|---|---|---|---|
| *^{a}* Step (b) of the present invention, wherein (±)-2-benzyloxycyclohexanone is obtained according to step (a) of the present invention. *^{b}* Determined by HPLC. *^{c}* The absolute configuration of the major stereoisomer is as that indicated between brackets in Table 5. The ee was >99% in all cases. | | | |

**Table 7. Enzymatic transamination of (±)-2-benzyloxycyclohexanone using a given ω-transaminase (ω-TA) at pH 11.5.^{a}**

| | | | | | |
|---|---|---|---|---|---|
| 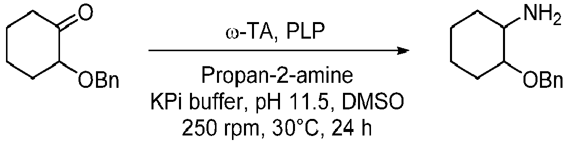 | | | | | |

| **ω-TA** | ***c*(%)*^{b}*** | ***dr*(%)***^{b}* | | ***ee*(%)***^{b}* | |
|---|---|---|---|---|---|
| | | ***cis*** | ***trans*** | ***cis*** | ***trans*** |
| ATA-013 | 33 | 91 | 9 | >99 (1*R*,2*S*) | - |
| ATA-024 | >99 | 57 | 43 | >99 (1*R*,2*S*) | >99 (1*R*,2*R*) |
| ATA-025 | >99 | 60 | 40 | >99 (1*R*,2*S*) | >99 (1*R*,2*R*) |
| ATA-303 | >99 | 4 | 96 | - | >99 (1*R*,2*R*) |
| ATA-412 | 62 | 91 | 9 | >99 (1*R*,2*S*) | - |
| ATA-415 | 29 | 89 | 11 | >99 (1*R*,2*S*) | >99 (1*R*,2*R*) |

| | | | | | |
|---|---|---|---|---|---|
| *^{a}* Step (b) of the present invention, wherein (±)-2-benzyloxycyclohexanone is obtained according to step (a) of the present invention. *^{b}* Determined by HPLC. | | | | | |

**Table 8. Enzymatic transamination of (±)-benzyl (2-oxocyclohexyl)carbamate using a given ω-transaminase (ω-TA) at pH 10. ^{a}**

| | | | | | |
|---|---|---|---|---|---|
| 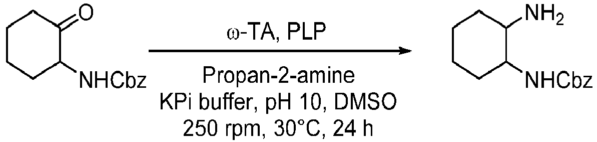 | | | | | |

| **ω-TA** | ***c*(%)*^{b}*** | ***dr*(%)***^{b}* | | ***ee*(%)***^{b}* | |
|---|---|---|---|---|---|
| | | ***cis*** | ***trans*** | ***cis*** | ***trans*** |
| ATA-238 | >99 | 42 | 58 | >99 (1*S*,2*R*) | >99 (1*S,*2*S*) |
| ATA-412 | >99 | 10 | 90 | >99 (1*R*,2*S*) | >99 (1*R,*2*R*) |
| ATA-415 | 97 | 63 | 37 | >99 (1*R*,2*S*) | >99 (1*R,*2*R*) |
| ATA-217 | >99 | 20 | 80 | >99 (1*S*,2*R*) | >99 (1*S,*2*S*) |
| ATA-024 | >99 | 28 | 72 | >99 (1*R*,2*S*) | >99 (1*R,*2*R*) |
| ATA-033 | >99 | 40 | 60 | >99 (1*R*,2*S*) | >99 (1*R,*2*R*) |

| | | | | | |
|---|---|---|---|---|---|
| *^{a}* Step (b) of the present invention, wherein (±)-2-benzyloxycyclohexanone is obtained according to step (a) of the present invention. *^{b}* Determined by HPLC. | | | | | |

### Example 4: Analytical methods

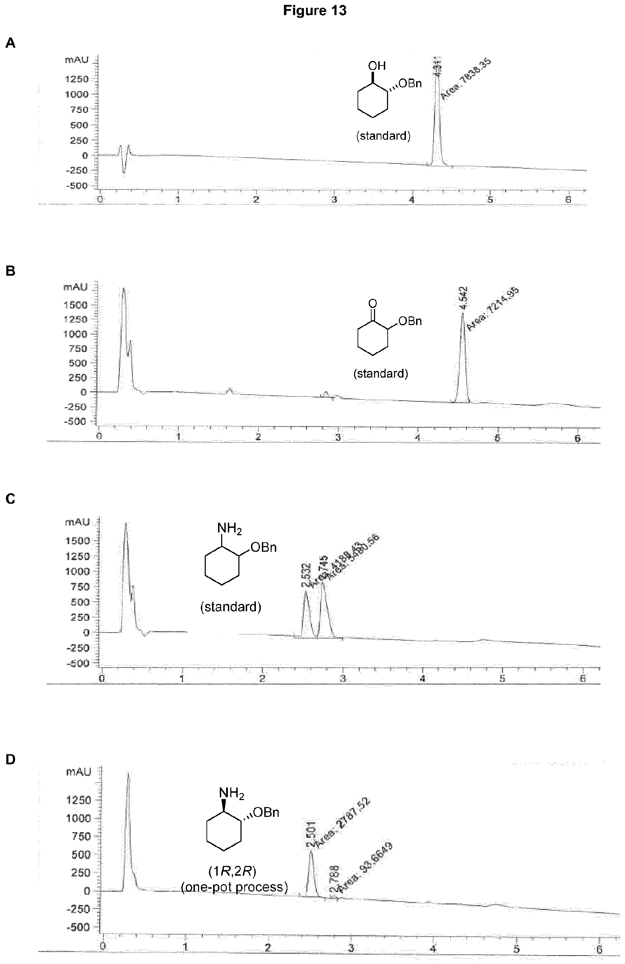

HPLC analyses for determine the conversion and diasteromeric ratio in the enzymatic amination of 2-(benzyloxy)cyclohexanone were carried out in an Agilent chromatographic system (Figure 13), using a reversed phase column (Zorbax Eclipse XDB-C18, RR, 1.8 µm, 4.6 x 50 mm, Agilent) and acetonitrile (MeCN) and 0.1% trifluoroacetic acid (TFA) in water as solvents. Samples were eluted with three linear gradients from 10% to 60% MeCN over 5.70 min, followed by another from 60% to 100% MeCN over 0.5 min and a third gradient from 100% to 10% MeCN over 1.90 min, at a flow rate of 2 mL/min. Detection and spectral characterization of peaks (UV absorption maxima (from HPLC-diode array) at 210 and 254 nm) were performed with a diode array detector and ChemStation Rev.B.03.01 software (Agilent).

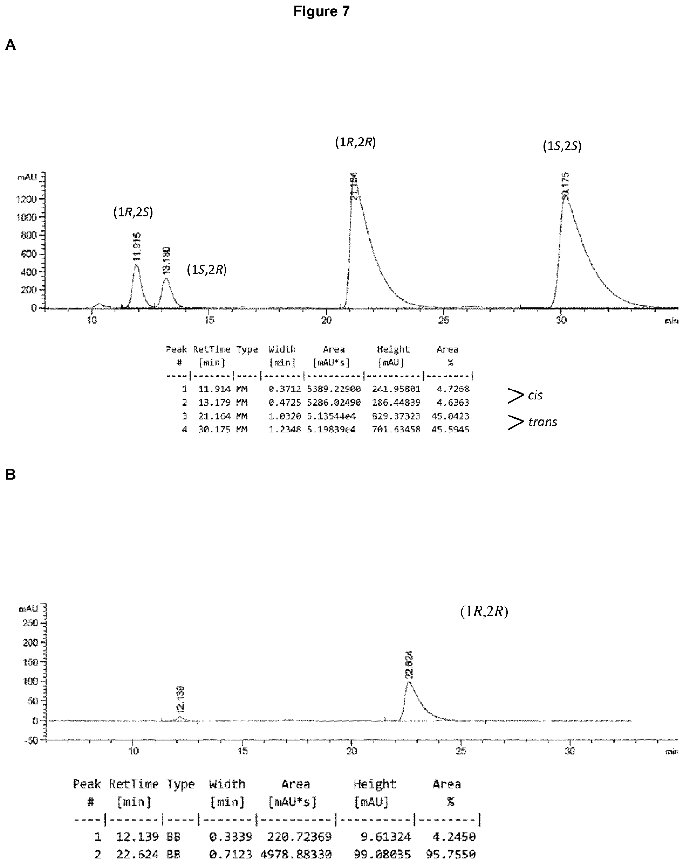

HPLC analyses for determine the enantiomeric excesses in the enzymatic amination of 2-(benzyloxy)cyclohexanone were carried out in an Agilent chromatographic system (Figure 7), using a column Chiralpak IA (25 cm × 4.6 mm I.D.) with a mixture of hexane:propan-2-ol (90:10), as eluent, at a flow rate of 0.8 mL/min and a temperature of 30 °C. Detection wavelength: 210 nm.

### Example 5: One-pot transamination of oxidation products of various acyclic and cyclic compounds comprising a hydroxyl group in the oxidation reaction mixture.

Following reaction of 1-phenylpropan-1-ol as per Example 1 for 1 h, the resulting reaction mixture was diluted 10 times with a 100 mM phosphate buffer of pH 7.5 (1.0 mL, comprising 1 M IPM) and DMSO (15% v/v), PLP (1 mM) and a given transaminase from Table 4 (5 mg, Codex^{®} Transaminase Screening Kit) was consecutively added. The mixture was shaken at 250 rpm and 30 °C for 12 h. The reaction was shaken at 250 rpm and 30 °C for 24 h. Then the mixture was extracted with ethyl acetate (2 × 3 mL), the organic layers separated by centrifugation (90 sec, 13000 rpm), combined and finally dried over Na₂SO₄. Conversion was determined directly by HPLC in reverse phase and the enantiomeric excess of the resulting 1-phenylpropan-1-amine measured by chiral HPLC after derivatization with di-*tert*-butyl dicarbonate.

This example illustrates that the transamination reaction is fully compatible with the conditions of the oxidation reaction mixture. With most of the transaminases from Codex^{®} Transaminase Screening Kit, 1-phenylpropan-2-one was converted to 1-phenylpropan-1-amine at a yield of more than 80% after 12 h of reaction time, more specifically 83% if ATA-251 was used, and >99% *ee* of the (*S*)-amine. Other acyclic compounds were oxidized and transaminated at a total combined yield >80% as shown in Table 9. The rate of conversion, enantiomeric excess and the identity of all the products were determined using HPLC and NMR.

The aforementioned sequential oxidation-transamination method was also performed substituting 1-phenylpropan-1-ol for any of the other substrates given in Table 9. The results for all such sequential oxidation-transamination reactions are presented in Table 9.

**Table 9. Sequential oxidation-transamination of alcohols mediated by 2-azaadamantane-N-oxyl (AZADO)/NaOCI and a transaminase.^{a}**

| | | | | | | |
|---|---|---|---|---|---|---|
| 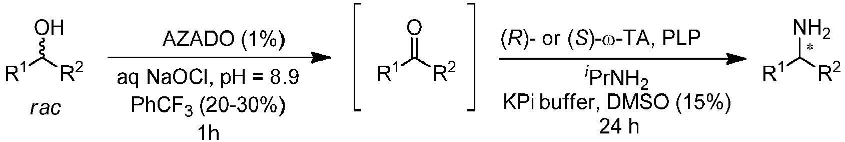 | | | | | | |

| **Substrate** | **ω-TA** | **Ratio(%)***^{b}* | | | ***trans:cis*** | ***ee*(%)***^{b}* |
|---|---|---|---|---|---|---|
| | | **Alcohol** | **Ketone** | **Amine** | | |
| 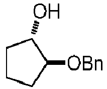 | ATA-303 | <1 | <1 | >99 | 86:14 | >99 (1*R,*2*R*)*^{c}* |
| | ArS | <1 | <1 | >99 | 73:27 | 94 (1*S,*2*S*)*^{c}* |
| | ATA-P2-A01 | <1 | <1 | >99 | 5:95 | >99 (1*R,*2*S*)*^{c}* |
| 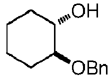 | ATA-303 | <1 | <1 | >99 | 96:4 | >99 (1*R,*2*R*)*^{c}* |
| | ArR | <1 | 42 | 58 | 5:95 | >99 (1*R,*2*S*)*^{c}* |
| 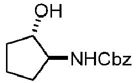 | ATA-024 | <1 | <1 | >99 | 80:20 | >99 (1*R,*2*R*)*^{c}* |
| 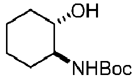 | ATA-238 | <1 | <1 | >99 | 94:6 | >99 (1*S,*2*S*)*^{c}* |
| 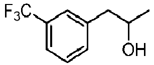 | ATA-P2-B01 | <1 | <1 | >99 | - | >99 (*R*) |
| | ArS | <1 | <1 | >99 | - | 92 (*S*) |
| 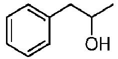 | ATA-251 | <1 | 4 | 96 | - | >99 (*S*) |
| | ArR | <1 | 9 | 91 | - | >99 (*R*) |
| 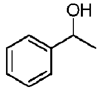 | ATA-P1-A06 | <1 | 20 | 80 | - | >99 (*S*) |
| 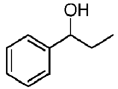 | ATA-033 | <1 | 13 | 87 | - | >99 (*R*) |
| 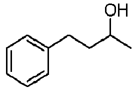 | ATA-P1-A06 | <1 | <1 | >99 | - | >99 (*S*) |
| | ArR | <1 | <1 | >99 | - | >99 (*R*) |
| 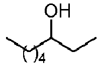 | ATA-033 | <1 | 15 | 85 | - | 97 (*R*) |

| | | | | | | |
|---|---|---|---|---|---|---|
| *^{a}* Oxidation step following the optimized conditions described in Table 1 for each substrate. Once completed, addition of ω-TA, DMSO (15% v/v) and KPi buffer (100 mM) at pH 7.5 (for acyclic substrates) or pH 11.5 (for cyclic substrates) with *ⁱ*Pr-NH₂ (1.0 M) PLP (1.0 mM) until a concentration of 20 mM and stirring at 250 rpm and 30 °C. *^{b}* Determined by GC or HPLC. *^{c} ee* and configuration of the major diastereomer. | | | | | | |

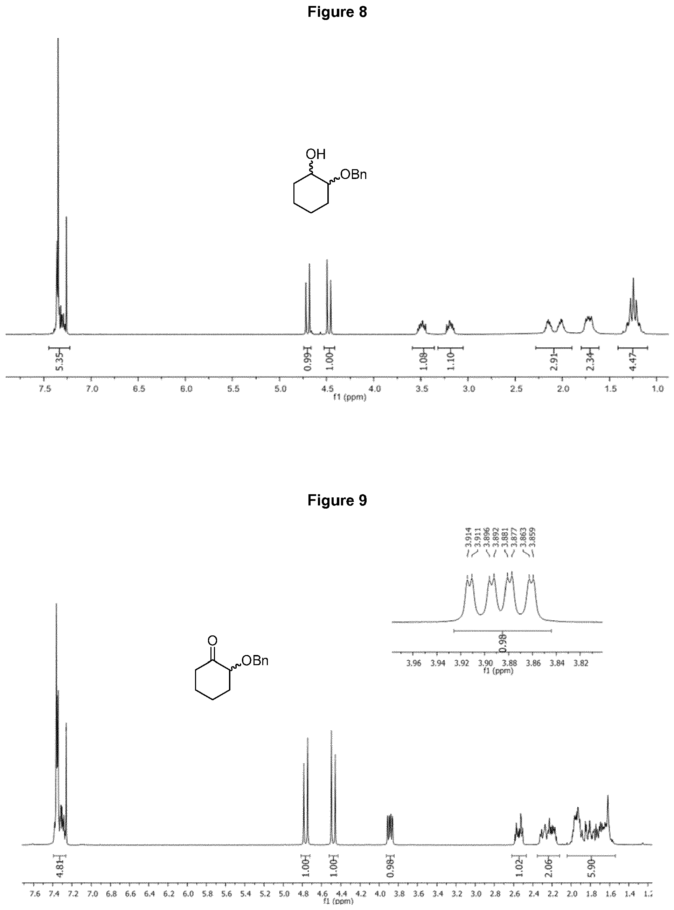

### (±)-trans-2-Benzyloxycyclohexanol (Figure 8)

¹H NMR (300 MHz, CDCl₃) δ (ppm): 1.17-1.35 (m, 4H), 1.67-1.75 (m, 2H), 1.98-2.17 (m, 3H), 3.15-3.22 (m, 1 H), 3.45-3.53 (m, 1 H), 4.45-4.49 (AB, 1 H, *J* 11.4 Hz), 4.68-4.72 (AB, 1 H, *J* 11.7 Hz), 7.27-7.38 (m, 5H).

### (±)-2-Benzyloxycyclohexanone (Figure 9)

¹H NMR (300 MHz, CDCl₃) δ (ppm): 1.61-1.97 (m, 5H), 2.15-2.32 (m, 2H), 2.50-2.58 (m, 1H), 3.86-3.91 (ddd, 1 H, *J* 0.9 Hz, *J* 5.4 Hz, *J* 9.9 Hz), 4.45-4.49 (AB, 1H, *J* 12 Hz), 4.74-4.78 (AB, 1 H, *J* 12 Hz), 7.28-7.38 (m, 5H); ¹³C NMR (300 MHz, CDCl₃) δ (ppm): 23.24 (CH₂), 27.76 (CH₂), 34.69 (CH₂), 40.77 (CH₂), 71.75 (CH₂), 81.80 (CH), 127.82 (CH*ₐᵣ*), 127.87 (2CH*ₐᵣ*), 128.51 (2CH*ₐᵣ*), 138.06 (C), 210.29 (CO).

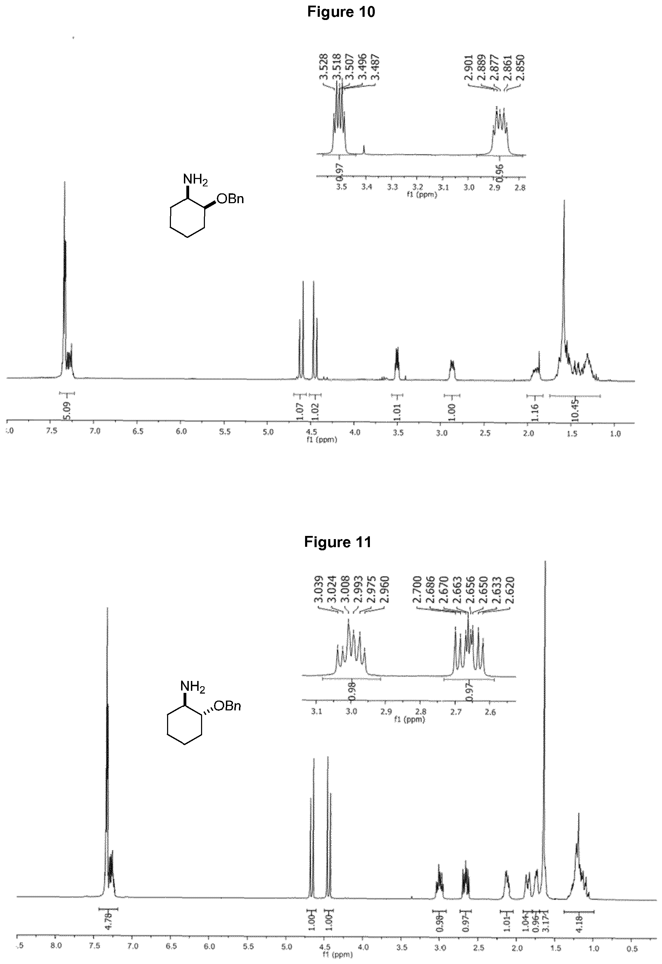

### (±)-cis-2-Benzyloxycyclohexylamine (Figure 10)

¹H NMR (300 MHz, CDCl₃) δ (ppm): 1.25-1.67 (m, 9H), 1.86-1.95 (m, 1 H), 2.85-2.90 (dt, 1 H, J 3.6 Hz (t); *J* 7.2 Hz (d)), 3.48-3.52 (dt, 1 H, *J* 3 Hz (t); *J* 6.3 Hz (d)); 4.43-4.47 (AB, 1 H, *J* 11.7 Hz), 4.59-4.63 (AB, 1 H, *J* 11.7 Hz), 7.23-7.36 (m, 5H); ¹³C NMR (300 MHz, CDCl₃) δ (ppm): 21.26 (CH₂), 23.04 (CH₂), 27.30 (CH₂), 31.41 (CH₂), 51.45 (C1), 70.33 (CH₂), 78.60 (C2), 127.49 (CH*ₐᵣ*), 127.57 (2CH*ₐᵣ*), 128.40 (2CH*ₐᵣ*), 139.19 (C).

### (±)-trans-2-Benzyloxycyclohexylamine (Figure 11)

¹H NMR (300 MHz, CDCl₃) δ (ppm): 1.05-1.82 (m, 4H), 1.60-1.67 (m, 3H), 1.72-1.76 (m, 1 H), 1.81-1.88 (m, 1 H), 2.09-2.16 (m, 1 H), 2.62-2.70 (ddd, 1H, *J₁* 4.2 Hz, *J₂* 9 Hz, *J₃* 11.1 Hz), 2.96-3.03 (td, 1 H, *J* 4.5 Hz (d); *J* 9.3 Hz (t)), 4.42-4.46 (AB, 1 H, *J* 11.4 Hz), 4.64-4.68 (AB, 1 H, *J* 11.4 Hz), 7.22-7.35 (m, 5H); ¹³C NMR (300 MHz, CDCl₃) δ (ppm): 24.64 (2CH₂), 29.84 (CH₂), 33.63 (CH₂), 55.16 (C1), 70.78 (CH₂), 84.61 (C2), 127.55 (CH*ₐᵣ*), 127.77 (2CH*ₐᵣ*), 128.39 (2CH*ₐᵣ*), 138.90 (C).

## Claims

1. A method for producing a compound of formula (III) 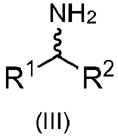 or a salt thereof, wherein said method comprises the following steps:
(a) forming a mixture comprising a compound of formula (II) 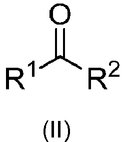 by adding an organocatalyst to a compound of formula (I) 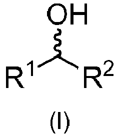 in an aqueous medium comprising an oxidizing agent and an organic co-solvent; and
(b) adding an aqueous buffer, a transamination catalyst, an amine donor compound and a cofactor to the mixture obtained in step (a), wherein said transamination catalyst is added at the same time as or after adding said aqueous buffer,
wherein:
(i) R¹ is an R³ substituent and R² is an R³ substituent, wherein each R³ substituent is independently selected from:
- an R^{3a} substituent;
- an R^{3b} substituent; or
- an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with
- an R^{3a} substituent,
- an R^{3b} substituent, or
- an R^{3a} substituent that is optionally substituted with an R^{3b} substituent,
or
(ii) R¹ and R², when taken with the carbon atom to which they are both attached, complete a non-aromatic 5-membered to 8-membered ring of a carbocycle or a heterocycle comprising at least one heteroatom selected from O, N or S, wherein said carbocycle or heterocycle comprises one ring or two to four fused 5-membered to 8-membered rings, and wherein said carbocycle or heterocycle is unsubstituted or substituted at one or more atoms of N, C or S with 1 to 4 R³ substituents, wherein each R³ substituent is independently selected from:
- an R^{3a} substituent;
- an R^{3b} substituent;
- an R^{3a} substituent which is substituted with 1 to 4 substituents each independently selected from =O, =NR⁴, =S, -NO₂, -N₃, -Z, an R^{3b} substituent or an R^{3a} substituent that is optionally substituted with an R^{3b} substituent;
- =O;
- =NR⁴; or
- =S,
and wherein:
· each R^{3a} substituent is independently selected from an alkyl, an alkenyl, an alkynyl, a carbocyclyl or a heterocyclyl moiety comprising at least one heteroatom selected from O, N or S, wherein said carbocyclyl or a heterocyclyl moiety comprises one to four fused 5-membered to 8-membered rings;
· each R^{3b} substituent is independently selected from -OR⁵, -(C=O)-R⁵, -(C=O)-OR⁵, -(C=O)-NR⁴R⁶, -O(C=O)-OR⁵, -NR^{C}(C=O)-OR⁵, -O(C=O)-NR⁴R⁶, -NR⁴R⁶, -CN,-Si(R⁵)₃, -SR⁵, -(S=O)-R⁵, -(S=NR⁴)-R⁵, -S(=O)₂-R⁵, -S(=O)₂-NR⁴R⁶ or -S(=O)(=NR⁴)-R⁵;
· R⁴ is H or R⁶;
· R⁵ is H or an R^{3a} substituent that is optionally substituted with another R^{3a} substituent;
· R⁶ is an R^{3a} substituent or -(C=O)-OR⁵; and
· Z is halogen.

2. The method according to claim 1, wherein the organocatalyst is selected from 2,2,6,6-tetramethylpiperidin-1-oxyl, 4-substituted derivatives of 2,2,6,6-tetramethylpiperidin-1-oxyl, 9-azabicyclo[3.3.1]nonane-*N-*oxyl, 9-azabicyclo[3,3,1]nonan-3-one-9-oxyl, 2,2'-azino-bis(3-ethylbenzothiazoline-6-sulphonic acid), violuric acid, 2-azaadamantane-*N-*oxyl, 1-methyl-2-azaadamantane-*N*-oxyl, 1,3-dimethyl-2-azaadamantane-*N-*oxyl or *N-*hydroxyphthalimide.

3. The method according to any of claims 1 to 2, wherein the oxidizing agent of step (a) is selected from NaClO, I₂, iodosylbenzene, 2-iodoxybenzoic acid, 1,1,1-triacetoxy-1,1-dihydro-1,2-benziodoxol-3(1H)-one, or a mixture of NaClO, KBr and B_{U4}NBr.

4. The method according to any of claims 1 to 3, wherein step (a) is carried out at a pH between 7 and 12.

5. The method according to any of claims 1 to 4, wherein the co-solvent of step (a) is selected from ethyl acetate, *tert-*butylmethyl ether, cyclopentylmethyl ether, *N,N-*dimethylformamide, tetrahydrofuran, 2-methyltetrahydrofuran, toluene, trifluorotoluene, dichloromethane or chloroform.

6. The method according to any of claims 1 to 5, wherein the transamination catalyst is a transaminase enzyme.

7. The method according to any of claims 1 to 6 wherein the amino donor compound comprises a primary amino moiety.

8. The method according to any of claims 1 to 7, wherein the cofactor is selected from the group consisting of pyridoxal-5'-phosphate (PLP), pyridoxine (PN), pyridoxal (PL), pyridoxamine (PM), pyridoxine phosphate (PNP) and pyridoxamine phosphate (PMP).

9. The method according to any of claims 1 to 8, wherein step (b) is carried out at a pH of at least 7.

10. The method according to any of claims 1 to 9, wherein step (b) additionally comprises adding a co-solvent selected from dimethyl sulfoxide, dimethyl acetamide, dimethyl formamide, acetonitrile, tetrahydrofuran, dioxane, an alcohol, ethylene glycol or an ether of ethylene glycol to the mixture obtained in step (a).

11. The method according to any of claims 1 to 10, wherein the compound of formula (III) is formed enriched in one enantiomer thereof.

12. The method according to any of claims 1 to 11, wherein the compound of formula (I) is a compound of formula (Ia), 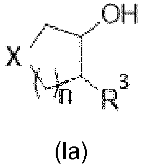 the compound of formula (II) is a compound of formula (IIa), 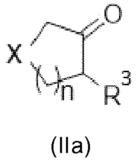 and the compound of formula (III) is a compound of formula (IIIa) 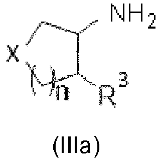 wherein
the compound of formula IIIa is formed enriched in one enantiomer and/or enriched in at least one diastereomer thereof,
n represents a whole number selected from 1, 2 or 3,
X is selected from CR⁵R⁵, CR⁵R⁶, O, SO₂, S, NH or NR⁴, and
(i) R³ is selected from:
- an R^{3a} substituent;
- an R^{3b} substituent;
- an R^{3a} substituent which is substituted with 1 or 2 substituents each independently selected from =O, -Z an R^{3b} substituent or an R^{3a} substituent that is optionally substituted with an R^{3b} substituent,
or alternatively
(ii) R³, when taken with the carbon atom to which it is attached and the n carbon atom adjacent thereto, completes a 5-membered to 7-membered ring of a carbocycle or a heterocycle comprising at least one N atom, wherein said ring is unsubstituted or substituted with 1 or 2 substituents independently selected from:
- an R^{3a} substituent;
- an R^{3b} substituent;
- an R^{3a} substituent which is substituted with 1 or 2 substituents each independently selected from =O, -Z an R^{3b} substituent or an R^{3a} substituent that is optionally substituted with an R^{3b} substituent,
and wherein:
· each R^{3a} substituent is independently selected from C₁-C₁₂ alkyl, a carbocyclyl or a heterocyclyl moiety comprising at least one N atom, wherein said carbocyclyl or a heterocyclyl moiety comprises one 5-membered to 8-membered ring or two fused 5-membered to 8-membered rings;
· each R^{3b} substituent is independently selected from -OR⁵, -(C=O)-R⁵, -(C=O)-OR⁵, -(C=O)-NR⁴R⁶, -O(C=O)-OR⁵, -NR^{C}(C=O)-OR⁵, -O(C=O)-NR⁴R⁶, -NR⁴R⁶, -CN or -Si(R^{3a})₃;
· each R⁴ substituent is independently selected from H or R⁶;
· each R⁵ substituent is independently selected from H or an R^{3a} substituent that is optionally substituted with another R^{3a} substituent;
· R⁶ is an R^{3a} substituent or -(C=O)-OR⁵; and
· Z is F, Cl or Br

13. The method according to claim 12 wherein step (b) is carried out at a pH of between 9 and 12 and the compound of formula (IIIa) is formed by dynamic kinetic resolution, and n represents a whole number selected from 1, 2 or 3,
X is selected from CH₂, CHR³ or O, and
each R³ is selected from:
- an R^{3a} substituent;
- an R^{3b} substituent;
- an R^{3a} substituent which is substituted with 1 or 2 substituents each independently selected from =O, an R^{3b} substituent or an R^{3a} substituent that is optionally substituted with an R^{3b} substituent,
wherein:
· each R^{3a} substituent is independently selected from a C₁-C₈ alkyl moiety, a carbocyclyl moiety which is a phenyl, or a heterocyclyl moiety comprising at least one N atom, wherein said heterocyclyl moiety comprises one or two fused 5-membered or 6-membered rings;
· each R^{3b} substituent is independently selected from -OR⁵, -(C=O)-OR⁵, -CN or-NH-(C=O)-OR⁵; and
· each R⁵ substituent is independently selected from H or an R^{3a} substituent that is optionally substituted with another R^{3a} substituent.

14. The method according to any of claims 1 to 13, wherein adding the buffer to the mixture obtained in step (a) dilutes said mixture at least two-fold.

15. The method according to any of claims 12 to 14 the compound of formula (III) is formed enriched in one diastereomer of:
(i) formula (IIIb1) or (IIIb2), 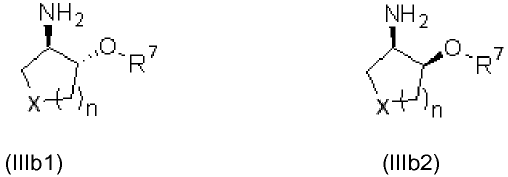 or a salt thereof; or
(ii) formula (IIIc1) or (IIIc2), 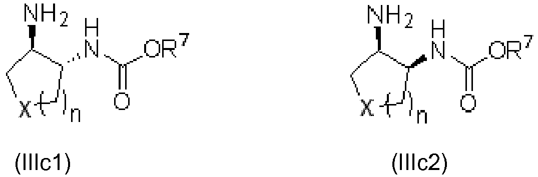 or a salt thereof,
wherein
n represents a whole number selected from 1, 2 or 3,
X is selected from CH₂ or O,
and R⁷ is selected from a benzyl or a *tert*-butyl moiety.
